(19) Europäisches Patentamt
European Patent Office
Office européen des brevets

(11) Veröffentlichungsnummer: **0 441 213 B1**

(12) **EUROPÄISCHE PATENTSCHRIFT**

(45) Veröffentlichungstag der Patentschrift: **13.09.95**

(51) Int. Cl.$^{6}$: **C07D 317/32**, C09K 19/58

(21) Anmeldenummer: **91101046.0**

(22) Anmeldetag: **28.01.91**

(54) **Chirale Dioxolane.**

(30) Priorität: **06.02.90 CH 371/90**
**19.10.90 CH 3344/90**

(43) Veröffentlichungstag der Anmeldung:
**14.08.91 Patentblatt 91/33**

(45) Bekanntmachung des Hinweises auf die Patenterteilung:
**13.09.95 Patentblatt 95/37**

(84) Benannte Vertragsstaaten:
**CH DE FR GB IT LI NL**

(56) Entgegenhaltungen:
**EP-A- 0 234 437**

(73) Patentinhaber: **F. HOFFMANN-LA ROCHE AG**
**Postfach 3255**
**CH-4002 Basel (CH)**

(72) Erfinder: **Kelly, Stephen, Dr.**
**Frankenstrasse 10**
**CH-4313 Möhlin (CH)**
Erfinder: **Schadt, Martin, Dr.**
**Liestalerstrasse 77**
**CH-4411 Seltisberg (CH)**

(74) Vertreter: **Cottong, Norbert A. et al**
**Grenzacherstrasse 124**
**Postfach 3255**
**CH-4002 Basel (CH)**

EP 0 441 213 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die vorliegende Erfindung betrifft neue chirale Dotierstoffe für Flüssigkristalle sowie flüssigkristalline Gemische, die solche Dotierstoffe enthalten und deren Verwendung für optische und elektro-optische Zwecke.

Flüssigkristallmaterialien für elektro-optische Anzeigevorrichtungen enthalten häufig einen oder mehrere optisch aktive Zusatzstoffe zur Induzierung einer chiralen Struktur. Beispielsweise wird zur Verwendung in Anzeigevorrichtungen mit verdrillt nematischer Struktur bevorzugt ein nematischer Flüssigkristall mit optisch aktivem Zusatzstoff dotiert, z.B. um eine Umkehr der Verdrillungsrichtung (reverse twist) in TN-Zellen (twisted-nematic) zu vermeiden oder um eine ausreichende Verdrillung in Zellen mit hoch verdrillter nematischer Struktur, wie STN-Zellen (super twisted-nematic), SBE-Zellen (super birefringence effect) oder OMI-Zellen (optical mode interference) zu erreichen. Ferner können cholesterische Flüssigkristalle für Phase-Change-Zellen vorzugsweise aus einem nematischen Basismaterial und einem oder mehreren optisch aktiven Dotierstoffen bestehen und ferroelektrische Flüssigkristalle für Anzeigevorrichtungen tungen auf der Basis von chiral getilteten smektischen Phasen können vorzugsweise aus einem Material mit getilteter smektischer Phase und einem oder mehreren optisch aktiven Dotierstoffen bestehen.

Die elektro-optischen Kennlinien von Flüssigkristall--Anzeigevorrichtungen sind temperaturabhängig, was insbesondere beim Multiplexbetrieb störend ist. Es ist jedoch bekannt, dass diese Temperaturabhängigkeit mindestens teilweise kompensiert werden kann durch Zusatz von chiralem Dotierstoff, welcher eine mit steigender Temperatur abnehmende Ganghöhe induziert. Eine solche inverse Temperaturabhängigkeit wurde nur für wenige Verbindungen gefunden. Solche Dotierstoffe sind beispielsweise die in EP-A-0 234 437 beschriebenen Weinsäurederivate. Eine inverse Temperaturabhängigkeit kann aber auch durch Verwendung von mindestens 2 chiralen Dotierstoffen erreicht werden, welche eine unterschiedliche relative Temperaturabhängigkeit aufweisen und unterschiedliche Verdrillungsrichtung induzieren (DE-A-2827471). Allerdings erfordert dies meist einen relativ hohen Anteil an chiralen Dotierstoffen.

Cholesterische Flüssigkristalle reflektieren Licht in einem Wellenlängenbereich, für den die Wellenlänge etwa gleich der Helixganghöhe ist. Die spektrale Breite dieses Reflexionslichtes kann durch geeignete Wahl des Flüssigkristalles variiert werden. Das reflektierte Licht ist vollständig zirkular polarisiert. Die Drehrichtung des reflektierten Lichts hängt vom Drehsinn der cholesterischen Helixstruktur ab. Das entgegengesetzt zirkular polarisierte Licht wird ungeschwächt transmittiert. Diese Eigenschaften können zur Herstellung von optischen Filtern, Polarisatoren, Analysatoren etc. ausgenutzt werden. Ferner wurden cholesterische Flüssigkristalle auch verschiedentlich für thermochrome Anwendungen und in kosmetischen Präparaten verwendet.

Cholesterische Flüssigkristalle für obige Anwendungen können vorzugsweise aus einem nematischen oder cholesterischen Basismaterial und einem oder mehreren chiralen Dotierstoffen bestehen, was eine einfache Einstellung der gewünschten Helixganghöhe erlaubt.

Um cholesterische Gemische mit einer Ganghöhe im Bereich der Wellenlänge des sichtbaren Lichts zu erreichen, sollten die chiralen Dotierstoffe ein möglichst hohes Verdrillungsvermögen aufweisen und in üblichen Flüssigkristallmaterialien gut löslich sein. Weiterhin sollten die chiralen Dotierstoffe eine ausreichende Stabilität besitzen, mit dem Mesophasetyp des Flüssigkristallmaterials gut kompatibel sein und den Mesophasenbereich nicht zu stark einschränken. Solche Eigenschaften wären auch für chirale Dotierstoffe zur Erzielung der eingangs genannten verdrillt nematischen Strukturen erwünscht, da ihr Anteil so niedrig gehalten werden könnte, dass die Eigenschaften des Flüssigkristallmaterials nur unwesentlich beeinflusst werden.

Die Erfindung betrifft optisch aktive Verbindungen der allgemeinen Formel

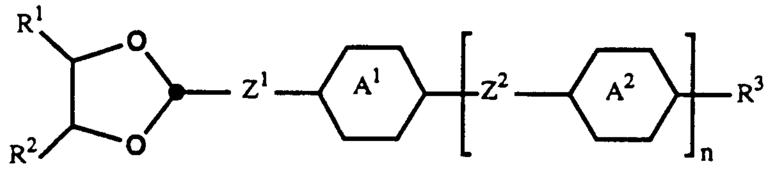

I

worin n für die Zahl 0 oder 1 steht; $R^3$ eine Gruppe der allgemeinen Formel

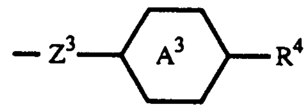

II

oder

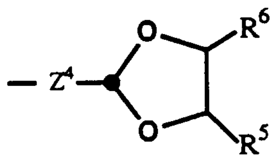

III

bezeichnet; die Ringe $A^1$, $A^2$ und $A^3$ unabhängig voneinander unsubstituiertes oder mit Halogen, Cyano und/oder Methyl substituiertes 1,4-Phenylen, in welchem gegebenenfalls 1 oder 2 CH-Gruppen durch Stickstoff ersetzt sind, trans-1,4-Cyclohexylen, trans-1,3-Dioxan-2,5-diyl oder Naphthalin-2,6-diyl darstellen und Ring $A^2$ zusätzlich 4,4'-Biphenylen darstellt; $Z^1$ und $Z^4$ unabhängig voneinander eine einfache Kovalenzbindung oder $-CH_2CH_2-$ bedeuten; $Z^2$ und $Z^3$ unabhängig voneinander eine einfache Kovalenzbindung, $-CH_2CH_2-$, -COO-, -OOC-, $-CH_2O-$, $-OCH_2-$, -C≡C-, $-(CH_2)_4-$, $-(CH_2)_3O-$, $-O(CH_2)_3-$ oder die trans-Form von $-CH=CH-CH_2CH_2-$, $-CH_2CH_2-CH=CH-$, $-CH=CH-CH_2O-$ oder $-OCH_2-CH=CH-$ bezeichnen; mit der Massgabe, dass, wenn $R^3$ eine Gruppe der Formel II bedeutet, mindestens eine der Gruppen $Z^2$ und $Z^3$ $-(CH_2)_4-$, $-(CH_2)_3-O-$, $-O-(CH_2)_3-$ oder die trans-Form von $-CH=CH-CH_2-CH_2-$, $-CH_2-CH_2-CH=CH-$, $-CH=CH-CH_2-O-$ oder $-O-CH_2-CH=CH-$ bedeutet; $R^1$, $R^5$ und $R^6$ Wasserstoff, Alkyl, Phenyl oder Alkoxycarbonyl bedeuten; $R^2$ Alkyl, Phenyl oder Alkoxycarbonyl bedeutet; $R^4$ Halogen, Cyano, Trifluormethyl, Trifluormethoxy oder Alkyl bezeichnet, in welchem gegebenenfalls eine $-CH_2-CH_2-$ durch $-CH=CH-$ ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist; X Halogen, Cyano oder Methyl bedeutet; und der in Formel I dargestellte Dioxolan-Ring in optisch aktiver Form vorliegt.

Die Verbindungen der Formel I sind sehr gut löslich in üblichen Flüssigkristallmaterialien und ermöglichen eine sehr hohe Verdrillung der Flüssigkristallstruktur. Im Unterschied zu bekannten Materialien mit hohen Verdrillungsvermögen werden zudem die Klärpunkte von Flüssigkristallen bei Zusatz von Verbindungen der Formel I in der Regel nicht oder nur unwesentlich gesenkt. Viele der erfindungsgemässen Verbindungen besitzen sogar selbst flüssigkristalline Eigenschaften. Die Verbindungen der Formel I sind leicht herstellbar, relativ niederviskos und ausreichend stabil gegen elektrische und magnetische Felder. Sie erfüllen daher in optimaler Weise die oben genannten Anforderungen.

Die Eigenschaften der Verbindungen der Formel I können je nach Zahl und Bedeutung der Ringe und der Substituenten in breiten Bereichen variiert werden. Beispielsweise führen aromatische Ringe zu höheren und gesättigte Ringe zu tieferen Werten der optischen Anisotropie. Eine Erhöhung des Klärpunktes kann beispielsweise durch Einführung eines weiteren Ringes erreicht werden. Polare Endgruppen, wie Cyano, Halogen, Trifluormethyl oder Trifluormethoxy, und Ringe, wie Pyrimidin-2,5-diyl oder trans-1,3-Dioxan-2,5-diyl, erhöhen die dielektrische Anisotropie; Ringe, wie 2,3-Dicyano-1,4-phenylen, vermindern die dielektrische Anisotropie; und laterale Halogen- und Cyanosubstituenten ergeben einen Beitrag zur Dielektrizitätskonstante sowohl parallel als auch senkrecht zur Moleküllängsachse, was je nach Substitutionsmuster zur Erhöhung oder Verminderung der dielektrischen Anisotropie ausgenützt werden kann. Ferner kann durch laterale Substituenten an einem oder mehreren Ringen eine allfällige Tendenz zur Ausbildung hochgeordneter smektischer Phasen oft weitgehend unterdrückt und oft auch die Löslichkeit verbessert werden. Weiterhin können durch eine C=C-Doppelbindung in der Seitenkette die elastischen Eigenschaften, die Schwellenspannungen, die Ansprechzeiten, die Mesophasen etc. weiter modifiziert werden.

Die vorliegende Erfindung ermöglicht daher neben der Induzierung einer hohen Verdrillung zusätzlich die Optimierung flüssigkristalliner und elektrooptischer Eigenschaften in einem weiten Bereich je nach Anwendung und gewünschten Eigenschaften.

Der Ausdruck "Halogen" bezeichnet im Rahmen der vorliegenden Erfindung Fluor, Chlor, Brom oder Jod, insbesondere Fluor oder Chlor.

Der Ausdruck "unsubstituiertes oder mit Halogen, Cyano und/oder Methyl substituiertes 1,4-Phenylen, in welchem gegebenenfalls 1 oder 2 CH-Gruppen durch Stickstoff ersetzt sind," umfasst Gruppen wie 1,4-Phenylen, 2-Fluor-1,4-phenylen, 2,3-Difluor-1,4-phenylen, 2-Chlor-1,4-phenylen, 2-Brom-1,4-phenylen, 2-Cyano-1,4-phenylen, 2,3-Dicyano-1,4-phenylen, 2-Methyl-1,4-phenylen, Pyridin-2,5-diyl, Pyrazin-2,5-diyl, Pyrimidin-2,5-diyl und dergleichen. Bevorzugte Gruppen sind 1,4-Phenylen, 2-Fluor-1,4-phenylen, 2,3-Difluor-1,4-phenylen, Pyridin-2,5-diyl und Pyrimidin-2,5-diyl.

Der Ausdruck "Alkyl" umfasst geradkettige und verzweigte, gegebenenfalls chirale Gruppen mit 1 bis 12 Kohlenstoffatomen, wie Methyl, Aethyl, Propyl, Isopropyl, Butyl, Isobutyl, 2-Butyl (=1-Methylpropyl), 2-Methylbutyl, Pentyl, Hexyl, Heptyl, Octyl, 2-Octyl (=1-Methylheptyl), Nonyl, Decyl, Undecyl, Dodecyl und dergleichen. Bevorzugte Alkylreste in $R^1$, $R^2$ , $R^5$ und $R^6$ sind $C_1$-$C_5$-Alkylreste, insbesondere Methyl.

Der Ausdruck "Alkoxycarbonyl" umfasst geradkettige und verzweigte, gegebenenfalls chirale Gruppen, wie Methoxycarbonyl, Aethoxycarbonyl, Propyloxycarbonyl, Isopropyloxycarbonyl, Butyloxycarbonyl, Isobutyloxycarbonyl, (2-Butyl)oxycarbonyl, (2-Methylbutyl)oxycarbonyl, Pentyloxycarbonyl, Hexyloxycarbonyl und dergleichen. Bevorzugte Alkoxycarbonylreste in $R^1$, $R^2$, $R^5$ und $R^6$ sind die $C_2$-$C_7$-Alkoxycarbonylreste, insbesondere die $C_2$-$C_5$-Alkoxycarbonylreste.

Der Ausdruck "Alkyl, in welchem gegebenenfalls eine $-CH_2-CH_2-$ durch $-CH=CH-$ ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist," umfasst geradkettige und verzweigte (gegebenenfalls chirale) Reste wie Alkyl, 1E-Alkenyl, 3E-Alkenyl, 4-Alkenyl, Alkenyl mit endständiger Doppelbindung, Alkoxy, 2E-Alkenyloxy, 3-Alkenyloxy, Alkenyloxy mit endständiger Doppelbindung, Alkoxyalkyl, Alkenyloxyalkyl, Alkoxycarbonyl, Alkoxycarbonylalkoxy (z.B. 1-(Alkoxycarbonyl)-1-äthoxy), Alkoxycarbonylalkoxycarbonyl (z.B. (1-Alkoxycarbonyl)-1-äthoxycarbonyl), Alkanoyloxy, 1-Haloalkyl, 2-Haloalkyl, 2-Haloalkoxy, 2-Haloalkoxycarbonyl, 1-Cyanoalkyl, 2-Cyanoalkyl, 2-Cyanoalkoxy, 2-Cyanoalkoxycarbonyl, 1-Methylalkyl, 2-Methylalkyl, 1-Methylalkoxy, 2-Methylalkoxy, 2-Methylalkoxycarbonyl und dergleichen. Beispiele bevorzugter Reste sind Methyl, Aethyl, Propyl, Butyl, Pentyl, Hexyl, Heptyl, Octyl, Nonyl, Decyl, Undecyl, Dodecyl, 1-Methylpropyl, 1-Methylheptyl, 2-Methylbutyl, 3-Methylpentyl, Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4Z-Heptenyl, 5-Hexenyl, 6-Heptenyl, 7-Octenyl, 8-Nonenyl, 9-Decenyl, 10-Undecenyl, 11-Dodecenyl, Methoxy, Aethoxy, Propyloxy, Butyloxy, Pentyloxy, Hexyloxy, Heptyloxy, Octyloxy, Nonyloxy, Decyloxy, Undecyloxy, Dodecyloxy, 1-Methylpropyloxy, 1-Methylheptyloxy, 2-Methylbutyloxy, 3-Methylpentyloxy, Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy, 7-Octenyloxy, 8-Nonenyloxy, 9-Decenyloxy, 10-Undecenyloxy, 11-Dodecenyloxy, Methoxymethyl, Aethoxymethyl, Propyloxymethyl, Allyloxymethyl, Methoxycarbonyl, Aethoxycarbonyl, Propyloxycarbonyl, 1-Methylpropyloxycarbonyl, 1-(Methoxycarbonyl)äthoxy, 1-(Aethoxycarbonyl)äthoxy, 1-(Methoxycarbonyl)-äthoxycarbonyl, 1-(Aethoxycarbonyl)äthoxycarbonyl, 1-(Isopropyloxycarbonyl)äthoxycarbonyl, 1-(Butyloxycarbonyl)äthoxycarbonyl, Acetoxy, Propionyloxy, Butyryloxy, 1-Fluorpropyl, 1-Fluorpentyl, 1-Chlorpropyl, 2-Fluorpropyl, 2-Fluorpentyl, 2-Chlorpropyl, 2-Fluorpropyloxy, 2-Fluorbutyloxy, 2-Fluorpentyloxy, 2-Fluorhexyloxy, 2-Chlorpropyloxy, Chlorbutyloxy, 2-Fluorpropyloxycarbonyl, 2-Fluorbutyloxycarbonyl, 2-Fluorpentyloxycarbonyl, 2-Fluor-3-methylbutyloxycarbonyl, 2-Fluor-4-methylpentyloxycarbonyl, 2-Chlorpropyloxycarbonyl, 1-Cyanopropyl, 1-Cyanopentyl, 2-Cyanopropyl, 2-Cyanopentyl, 2-Cyanopropyloxy, 2-Cyanobutyloxy, 2-Cyanopentyloxy, 2-Cyanohexyloxy, 2-Cyanopropyloxycarbonyl, 2-Cyanobutyloxycarbonyl, 2-Cyano-3-methylbutyloxycarbonyl, 2-Cyano-4-methylpentyloxycarbonyl und dergleichen.

Formel I umfasst optisch aktive Verbindungen der folgenden allgemeinen Formeln

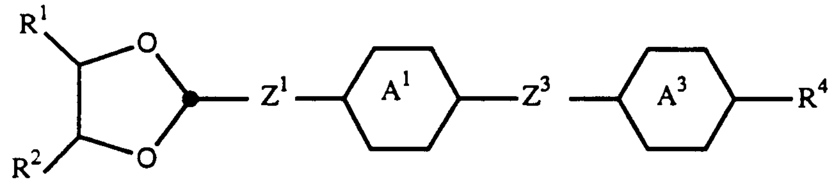

I-1

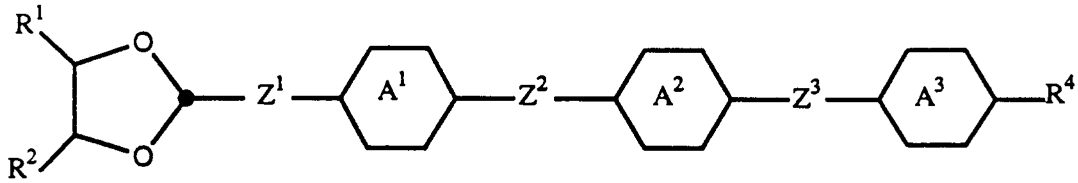

I-2

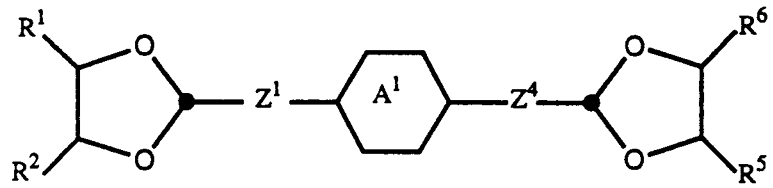

I-3

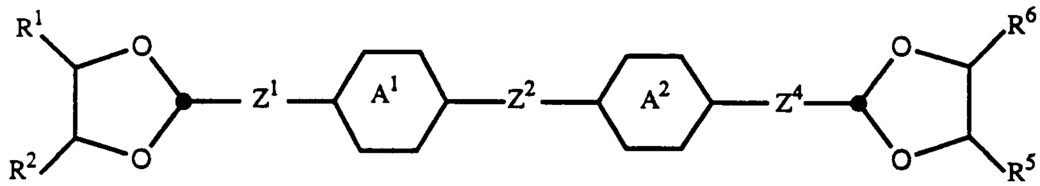

I-4

worin $R^1$, $R^2$, $R^4$, $R^5$, $R^6$, $A^1$, $A^2$, $A^3$, $Z^1$, $Z^2$, $Z^3$, $Z^4$ jeweils die oben genannten Bedeutungen haben, mit der Massgabe, dass bei den Verbindungen der Formeln I-1 und I-2 mindestens eine der Gruppen $Z^2$ und $Z^3$ $-(CH_2)_4-$, $-(CH_2)_3-O-$, $-O-(CH_2)_3-$ oder die trans-Form von $-CH=CH-CH_2-CH_2-$, $-CH_2-CH_2-CH=CH-$, $-CH=CH-CH_2-O-$ oder $-O-CH_2-CH=CH-$ bedeutet

Die Verbindungen der Formeln I-3 und I-4 sind in der Regel chirale Dotierstoffe mit besonders hohem Verdrillungsvermögen.

Die Verbindungen der Formel I besitzen mindestens einen optisch aktiven Dioxolan-Ring. Der in Formel I dargestellte Dioxolan-Ring besitzt in Position 4 (d.h. benachbart zu $R^2$) ein chirales Kohlenstoffatom und, falls $R^1$ von Wasserstoff verschieden ist, in Position 5 (d.h. benachbart zu $R^1$ ein weiteres chirales Kohlenstoffatom. Es ist dem Fachmann klar, dass zur Erzielung optischer Aktivität das Kohlenstoffatom in Position 4 des Dioxolan-Ringes ganz oder überwiegend in R- oder S-Form vorliegen muss und, falls $R^1$ und $R^2$ identisch sind, das Kohlenstoffatom in Position 5 des Dioxolan-Ringes ganz oder überwiegend die gleiche Konfiguration aufweisen sollte wie das Kohlenstoffatom in Position 4 oder grundsätzlich auch ein R/S-Verhältnis von 50:50 aufweisen kann. Um ein möglichst hohes Verdrillungsvermögen zu erzielen sollte das Kohlenstoffatom in Position 4 des Dioxolan-Ringes vorzugsweise in optisch möglichst reiner Form in R- oder S-Konfiguration vorliegen und, falls $R^1$ von Wasserstoff verschieden ist, das Kohlenstoffatom in Position 5 des Dioxolan-Ringes vorzugsweise in optisch möglichst reiner Form in jener Konfiguration vorliegen, die das Verdrillungsvermögen verstärkt. Bevorzugte optische Isomere mit hohen Verdrillungsvermögen sind die (4R)-Isomere und die (4S)-Isomere der Verbindungen der Formel I, worin $R^1$ Wasserstoff bedeutet, und die (4R,5R)-Isomere und die (4S,5S)-Isomere der Verbindungen der Formel I, worin $R^1$ Alkyl, Phenyl oder Alkoxycarbonyl bedeutet.

Besonders bevorzugt sind im allgemeinen diejenigen Verbindungen der Formeln I, I-1 und I-2, worin $A^1$, $A^2$ und $A^3$ unabhängig voneinander unsubstituiertes oder mit Halogen, Cyano und/oder Methyl substituiertes 1,4-Phenylen (insbesonderes 1,4-Phenylen, 2-Fluor-1,4-phenylen oder 2,3-Difluor-1,4-phenylen) oder trans-1,4-Cyclohexylen darstellen oder eine der Gruppen $A^1$, $A^2$ und $A^3$ auch Pyrimidin-2,5-diyl darstellt, und ferner eine der Gruppen $Z^2$ und $Z^3$ $-(CH_2)_4-$, $-O-(CH_2)_3-$, $-(CH_2)_3-O-$oder die trans-Form von $-CH_2-CH_2-$

$CH=CH$-, $-CH=CH-CH_2-CH_2-$, $-O-CH_2-CH=CH-$ oder $-CH=CH-CH_2-O-$bezeichnet und, falls vorhanden, die andere der Gruppen $Z^2$ und $Z^3$ eine einfache Kovalenzbindung bezeichnet. Diese Verbindungen sind sehr stabil und in der Regel besonders leicht zugänglich aus bekannten Zwischenprodukten von Flüssigkristallen.

Ganz besonders bevorzugt sind diejenigen Verbindungen der Formeln I-3 und I-4, worin die Ringe $A^1$ und $A^2$ unabhängig voneinander unsubstituiertes oder mit Halogen, Cyano und/oder Methyl substituiertes 1,4-Phenylen (insbesondere 1,4-Phenylen, 2-Fluor-1,4-phenylen oder 2,3-Difluorphenylen) oder trans-1,4-Cyclohexylen darstellen oder der Ring $A^2$ auch Pyrimidin-2,5-diyl oder 4,4'-Biphenylen darstellt und ferner eine gegebenenfalls vorhandene Gruppe $Z^2$ eine einfache Kovalenzbindung, $-CH_2-CH_2-$, -COO-, -OOC- (insbesondere eine einfache Kovalenzbindung) bezeichnet.

Da das Verdrillungsvermögen der erfindungsgemässen Verbindungen hauptsächlich durch den optisch aktiven Dioxolan-Rest bestimmt wird, ist jedoch klar, dass eine oder mehrere dieser besonders bevorzugten Ringe und Brückengruppen durch die weiteren in Formel I für $A^1$, $A^2$ und $A^3$ genannten Ringe und für $Z^1$, $Z^2$, $Z^3$ und $Z^4$ genannten Brückengruppen ersetzt werden können, um einen ähnlichen Effekt und ebenfalls gute Kompatibilität mit üblichen Flüssigkristallen zu erhalten. Im allgemeinen sind jedoch solche Verbindungen der Formeln I und I-1 bis I-4 bevorzugt, worin jeweils einer der Ringe $A^1$, $A^2$ und $A^3$ unsubstituiertes oder mit Halogen, Cyano und/oder Methyl substituiertes 1,4-Phenylen oder Pyridin-2,5-diyl, Pyrimidin-2,5--diyl, trans-1,4-Cyclohexylen, trans-1,3-Dioxan-2,5-diyl oder Naphthalin-2,6-diyl und Ring $A^2$ zusätzlich auch 4,4'-Biphenylen darstellt, die andern der Ringe $A^1$, $A^2$ und $A^3$ (falls vorhanden) unabhängig voneinander unsubstituiertes oder mit Halogen, Cyano und/oder Methyl substituiertes 1,4-Phenylen (insbesondere unsubstituiertes 1,4-Phenylen) oder trans-1,4-Cyclohexylen darstellen, eine der Gruppen $Z^2$ und $Z^3$ eine einfache Kovalenzbindung, $-CH_2CH_2-$, -COO-, -OOC-, $-CH_2O-$, $-OCH_2-$, -C≡C-, $-(CH_2)_4-$, $-(CH_2)_3O-$, $-O(CH_2)_3-$ oder die trans-Form von $-CH=CH_2-CH_2CH_2-$, $-CH_2CH_2-CH=CH-$, $-CH=CH-CH_2O-$ oder $-OCH_2-CH=CH-$ bezeichnet, und die andere der Gruppen $Z^2$ und $Z^3$ (falls vorhanden) eine einfache Kovalenzbindung bezeichnet, mit der Massgabe, dass, wenn $R^3$ eine Gruppe der Formel III bedeutet, mindestens eine der Gruppen $Z^2$ und $Z^3$ $-(CH_2)_4-$, $-(CH_2)_3-O-$, $-O-(CH_2)_3-$ oder die trans-Form von $-CH=CH-CH_2-CH_2-$, $-CH_2-CH_2-CH=CH-$, $-CH=CH-CH_2-O-$ oder $-O-CH_2-CH=CH-$ bedeutet.

Beispiele besonders bevorzugter Verbindungen der Formel I sind die optisch aktiven Verbindungen der folgenden allgemeinen Formeln

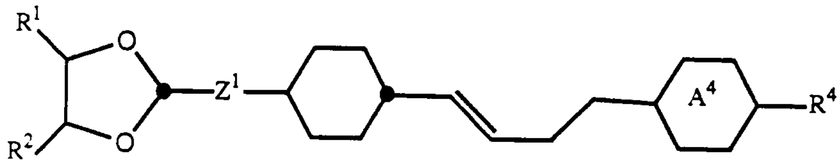

I-5

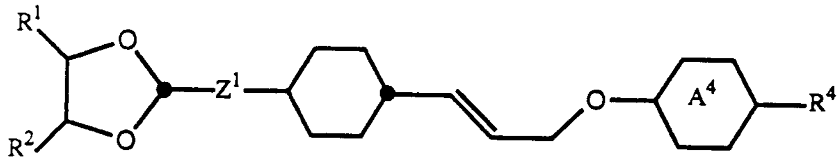

I-6

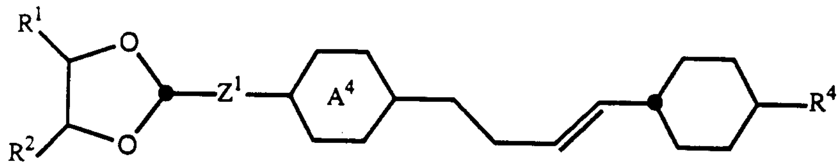

I-7

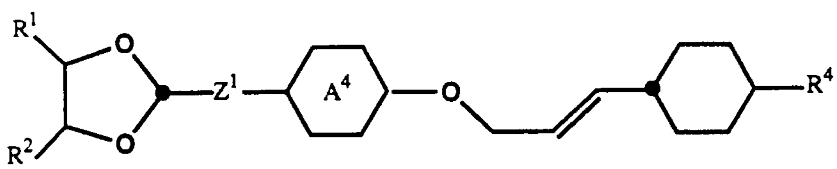

I-8

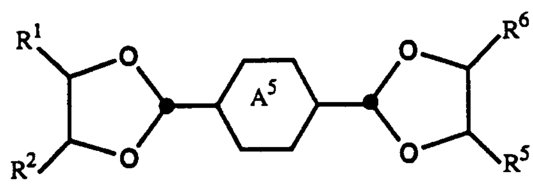

I-9

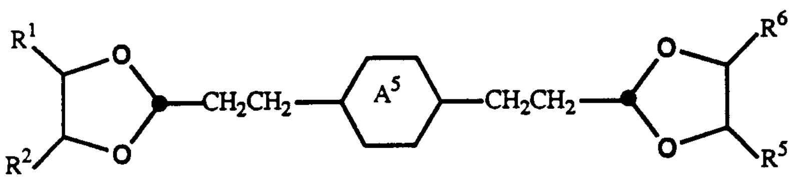

I-10

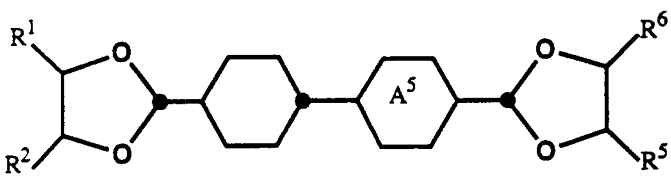

I-11

worin $R^1$, $R^2$, $R^4$, $R^5$ und $R^6$ die obigen Bedeutungen haben, und die Ringe $A^4$ und $A^5$ unsubstituiertes oder mit Halogen, Cyano und/oder Methyl substituiertes 1,4-Phenylen (vorzugsweise 1,4-Phenylen, 2-Fluor-1,4-phenylen oder 2,3-Difluor-1,4-phenylen) oder trans-1,4-Cyclohexylen darstellen und Ring $A^5$ zusätzlich 4,4'-Biphenylen bedeutet.

In den obigen Formeln I, I-1, I-2 und I-5 bis I-8 kann $R^4$ jeweils vorzugsweise Halogen (insbesondere Fluor oder Chlor), Cyano, Trifluormethyl, Trifluormethoxy, Alkyl, Alkenyl, Alkoxy oder Alkenyloxy bezeichnen. In einem Alkylrest $R^4$ können jedoch gewünschtenfalls auch eine oder zwei nicht benachbarte Methylengruppen durch -O-, -COO- und/oder -OOC- ersetzt und/oder eine Methylengruppe durch -CHX- (worin X Halogen, Cyano oder Methyl bedeutet) ersetzt werden. Diese Möglichkeit lässt sich z.B. ausnutzen zur Erhöhung des Verdrillungsvermögen durch Einführung chiraler Gruppen ausgehend von einfachen, optisch aktiven Verbindungen. Bevorzugte Beispiele derartiger Gruppen sind die von optisch aktiver Milchsäure abgeleiteten Reste, wie 1-(Alkoxycarbonyl)äthoxy und 1-(Alkoxycarbonyl)äthoxycarbonyl. $R^4$ in den obigen Formeln kann daher vorzugsweise jeweils auch einen dieser Reste bedeuten.

$R^4$ besitzt in der Regel 1 bis 18 Kohlenstoffatom. Im allgemeinen sind Reste $R^4$ mit 1 bis 12 Kohlenstoffatomen, insbesondere diejenigen mit 1 bis 7 Kohlenstoffatomen bevorzugt.

Ganz besonders bevorzugte Verbindungen der Formel I, worin $R^3$ eine Gruppe der Formel III ist, sind die Verbindungen der allgemeinen Formeln

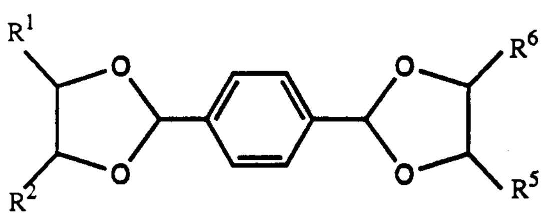

I-12

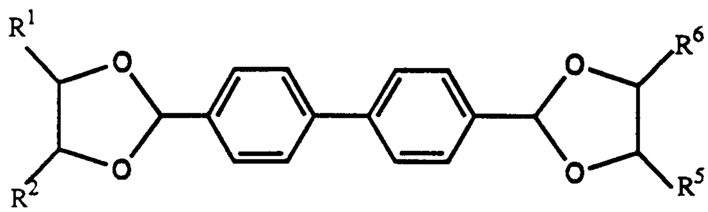

I-13

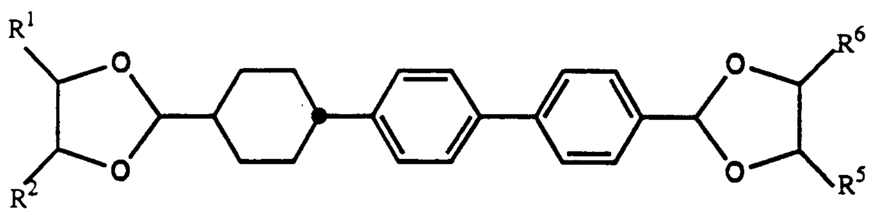

I-14

worin $R^1$, $R^2$, $R^5$ und $R^6$ die oben angegebenen Bedeutungen haben.

Die Verbindungen der Formel I können ferner eine Endgruppe der Formel III, d.h. einen weiteren Dioxolan-Rest aufweisen. Diese Gruppe kann grundsätzlich optisch inaktiv sein. Bevorzugt sind jedoch Gruppen der Formel III, welche in einer optisch aktiven Form vorliegen, die das Verdrillungsvermögen verstärkt. Besonders leicht herstellbare Verbindungen mit erhöhtem Verdrillungsvermögen sind diejenigen, worin beide Dioxolan-Reste die gleiche Konfiguration und die gleiche Reste aufweisen. Vorzugsweise kann daher in Formel III $R^5$ die gleiche Bedeutung und am benachbarten C-Atom die gleiche Konfiguration wie $R^1$ besitzen und $R^6$ die gleiche Bedeutung und am benachbarten C-Atom die gleiche Konfiguration wie $R^2$ besitzen. Dies gilt sinngemäss auch für die beiden Dioxolan-Reste in den Formeln I-3, I-4, I-12, I-13 und I-14.

Bevorzugte optische Isomere sind somit die Verbindungen mit der in der folgenden allgemeinen Formel angegebenen relativen Konfiguration:

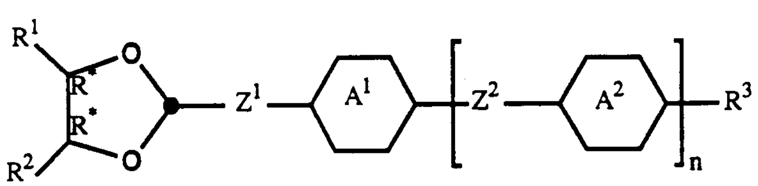

IA

worin $R^3$ eine Gruppe der allgemeinen Formel

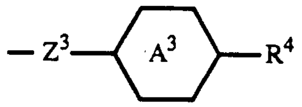

II

oder

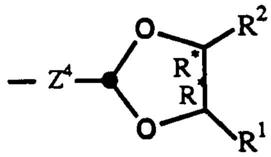

IIIA

bezeichnet; n, $A^1$, $A^2$, $A^3$, $Z^1$, $Z^2$, $Z^3$, $Z^4$, $R^1$, $R^2$ und $R^4$ die obigen Bedeutungen haben, mit der Massgabe, dass, wenn $R^3$ eine Gruppe der Formel II bedeutet, mindestens eine der Gruppen $Z^2$ und $Z^3$ $-(CH_2)_4-$, $-(CH_2)_3-O-$, $-O-(CH_2)_3-$ oder die trans-Form von $-CH=CH-CH_2-CH_2-$, $-CH_2-CH_2-CH=CH-$, $-CH=CH-CH_2-O-$ oder $-O-CH_2-CH=CH-$ bedeutet; und mit $R^*$ bezeichnete, asymmetrische Kohlenstoffatome alle in der R-Konfiguration oder alle in der S-Konfiguration vorliegen.

Formel IA umfasst die mit R angegebenen optischen Isomere sowie deren optische Antipoden, welche das gleiche Verdrillungsvermögen aber mit entgegengesetzter Verdrillungsrichtung besitzen.

Bevorzugte, optisch aktive Verbindungen der Formel IA sind diejenigen, worin $R^1$ und $R^2$ verschiedene oder vorzugsweise gleiche Bedeutung haben und $C_2$-$C_7$-Alkoxycarbonyl, insbesondere $C_2$-$C_5$-Alkoxycarbonyl bezeichnen. Diese Verbindungen sind leicht zugänglich aus den entsprechenden, optisch aktiven Weinsäuren bzw. Weinsäure-dialkylestern. Besonders bevorzugt sind diejenigen Verbindungen der Formeln I-1 bis I-14, worin jeweils $R^1$ und $R^2$ verschiedene oder vorzugsweise gleiche Bedeutung haben und $C_2$-$C_7$-Alkoxycarbonyl (insbesondere $C_2$-$C_5$-Alkoxycarbonyl) bezeichnen, $R^5$ die Bedeutung von $R^1$ hat, $R^6$ die Bedeutung von $R^2$ hat, und die asymmetrischen Kohlenstoffatome die in Formel IA angegebene relative Konfiguration aufweisen.

Weitere bevorzugte, optisch aktive Verbindungen der Formel IA sind diejenigen, worin $R^2$ Methyl oder Phenyl bezeichnet und $R^1$ die Bedeutung von $R^2$ hat oder Wasserstoff bedeutet. Diese Verbindungen sind leicht zugänglich aus den entsprechenden, optisch aktiven Diolen, nämlich 2,3-Butandiol, 1,2-Diphenyl-1,2-äthandiol, 1,2-Propandiol oder 1-Phenyl-1,2-äthandiol. Besonders bevorzugt sind diejenigen Verbindungen der Formeln I-1 bis I-14, worin jeweils $R^2$ Methyl oder Phenyl bezeichnet, $R^1$ die Bedeutung von $R^2$ hat oder Wasserstoff bedeutet, $R^5$ die Bedeutung von $R^1$ hat, $R^6$ die Bedeutung von $R^2$ hat und die asymmetrischen Kohlenstoffatome die in Formel IA angegebene relative Konfiguration aufweisen.

In obiger Formel IA und in den oben als bevorzugt genannten Untergruppen von Verbindungen der Formel I können Ring $A^1$, $A^2$, $A^3$, $A^4$ und $A^5$, $Z^1$, $Z^2$, $Z^3$, $Z^4$ insbesondere jeweils die im Zusammenhang mit den Formeln I und I-1 bis I-14 angegebenen, bevorzugten Bedeutungen haben, mit der Massgabe, dass, wenn $R^3$ eine Gruppe der Formel II bedeutet, mindestens eine der Gruppen $Z^2$ und $Z^3$ $-(CH_2)_4-$, $-(CH_2)_3-O-$, $-O-(CH_2)_3-$ oder die trans-Form von $-CH=CH-CH_2-CH_2-$, $-CH_2-CH_2-CH=CH-$, $-CH=CH-CH_2-O-$ oder $-O-CH_2-CH=CH-$ bedeutet.

Die erfindungsgemässen Verbindungen können beispielsweise dadurch hergestellt werden, dass man einen Aldehyd der Formel OHC-$Z^1$ $\Lambda^1$ $(Z^2-A^2)_n-R^3$ oder ein Acetal hiervon mit einem optisch aktiven Diol der Formel $R^1$-CH(OH)-CH(OH)-$R^2$ umsetzt. Die Umsetzung des Aldehyds oder eines geeigneten Acetals (z.B. des Dimethylacetals) mit dem Diol kann in an sich bekannter Weise erfolgen. Zweckmässigerweise erfolgt die Umsetzung in einem inerten organischen Lösungsmittel (beispielsweise einem aromatischen Kohlenwasserstoff, wie Benzol, Toluol oder Xylol) in Gegenwart einer katalytischen Menge einer organischen oder anorganischen Säure, wie p-Toluolsulfonsäure, Schwefelsäure oder trockenem Chlorwasserstoff. Temperatur und Druck sind nicht kritisch, vorzugsweise wird jedoch bei Rückflusstemperatur (unter Abtrennung des gebildeten Wassers) und Atmosphärendruck gearbeitet.

Ein gegebenenfalls vorhandener, zweiter Dioxolan-Ring (wenn $R^3$ eine Gruppe der Formel III bedeutet) kann in analoger Weise ebenfalls aus Aldehyd (bzw. Acetal) und Diol gebildet werden. Sind beide Diole [$R^1$-CH(OH)-CH(OH)-$R^2$ und $R^5$-CH(OH)-CH(OH)-$R^6$] identisch, können gewünschtenfalls beide Dioxolan-Ringe in einer Stufe gebildet werden.

Enthält die Verbindung der Formel I eine oder mehrere Estergruppen in $Z^2$, $Z^3$ und/oder $R^4$, so kann vorzugsweise die betreffende Veresterung auch erst nach der Bildung des Dioxolan-Ringes erfolgen. Gewünschtenfalls können auch Aethergruppen und andere funktionelle Gruppen erst nach der Bildung des Dioxolan-Ringes eingeführt werden.

Derartige Methoden sind dem Fachmann grundsätzlich bekannt, z.B. aus der Herstellung flüssigkristalliner Dioxane. Die zur Herstellung benötigten Diole sind bekannte oder Analoge bekannter Verbindungen. Die als Ausgangsmaterialien benötigten Aldehyde sind ebenfalls bekannte Verbindungen oder können nach an sich bekannten Methoden hergestellt werden. Derartige Verbindungen und Methoden sind z.B. bekannt aus EP-A-0115897, EP-A-0113459 und EP-A-0106991.

Die Erfindung betrifft ebenfalls ein flüssigkristallines Gemisch enthaltend ein flüssigkristallines Trägermaterial und eine oder mehrere optisch aktive Verbindungen der Formel I. Als Trägermaterial eignen sich grundsätzlich alle Flüssigkristallmaterialien, welche eine verdrillbare Flüssigkristallphase mit einem adäquaten Mesophasenbereich aufweisen. Besonders geeignet sind die Verbindungen der Formel I als chirale Dotierstoffe für nematische oder cholesterische Trägermaterialien. Das flüssigkristalline Trägermaterial kann eine Einzelverbindung oder ein Gemisch sein und besitzt vorzugsweise einen Klärpunkt von mindestens etwa 60 °C.

Der Anteil an chiralem Dotierstoff der Formel I ist im wesentlichen durch dessen Verdrillungsvermögen und die gewünschte Ganghöhe bestimmt. Der Anteil an chiralem Dotierstoff kann daher je nach Anwendung in einem breiten Bereich variieren und beispielsweise etwa 0,1-30 Gew.% betragen. Für Anzeigevorrichtungen auf der Basis von Flüssigkristallen mit verdrillt nematischer Struktur ist je nach Zellentyp und -dicke meist eine Ganghöhe von etwa 3-40 µm erforderlich und daher ein entsprechend kleiner Anteil ausreichend. Demgegenüber werden für Anwendungen, welche auf der Reflexion von sichtbarem Licht durch cholesterische Schichten beruhen, Ganghöhen von weniger als 2 µm, beispielsweise etwa 0,4-0,6 µm benötigt, was einen entsprechend höheren Anteil an chiralem Dotierstoff erfordert.

Geeignete flüssigkristalline Trägermaterialien sind in grosser Zahl bekannt und im Handel erhältlich. In der Regel sind flüssigkristalline Gemische enthaltend 2 oder mehrere Komponenten als Trägermaterialien bevorzugt. Grundsätzlich kann aber auch eine flüssigkristalline Verbindung als Trägermaterial verwendet werden, wenn sie eine ausreichend breite Mesophase aufweist.

Als Komponenten für flüssigkristalline Trägermaterialien eignen sich insbesondere Verbindungen der folgenden allgemeinen Formeln

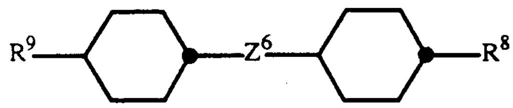

IV

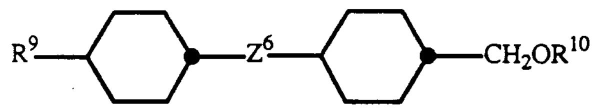

V

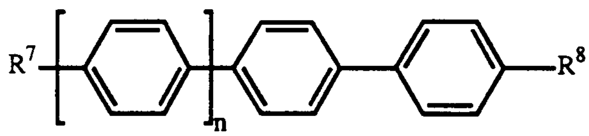

VI

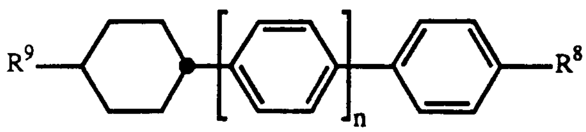

VII

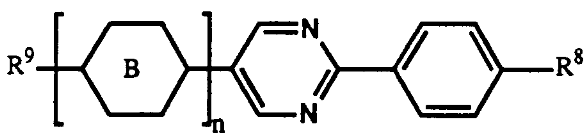

VIII

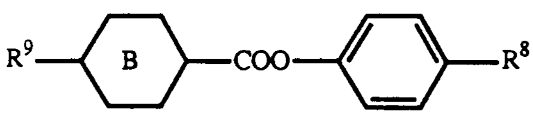

IX

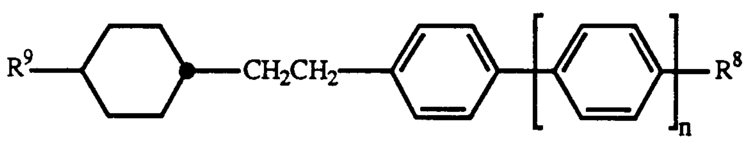

X

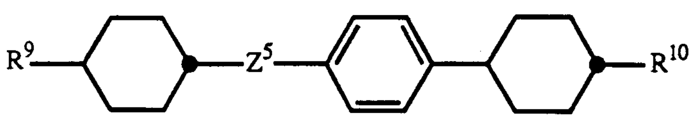

XI

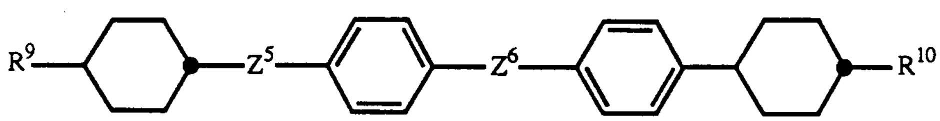

XII

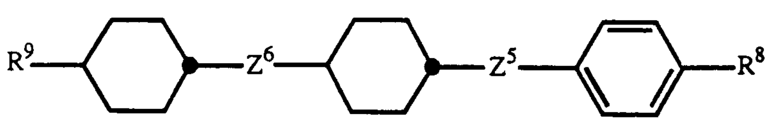

XIII

worin n für die Zahl 0 oder 1 steht; $R^7$ Alkyl, Alkoxy, Alkenyl oder Alkenyloxy bezeichnet; $R^8$ Cyano, Alkyl, Alkoxy, Alkenyl oder Alkenyloxy bedeutet; $R^9$ und $R^{10}$ unabhängig voneinander Alkyl oder Alkenyl bezeichnen; Ring B 1,4-Phenylen oder trans-1,4-Cyclohexylen darstellt; $Z^5$ eine einfache Kovalenzbindung, -COO- oder $-CH_2CH_2-$ bezeichnet; und $Z^6$ eine einfache Kovalenzbindung oder $-CH_2CH_2-$ bedeutet.

$R^7$, $R^8$, $R^9$ und $R^{10}$ besitzen vorzugsweise höchstens je 12 Kohlenstoffatome, besonders bevorzugt höchstens je 7 Kohlenstoffatome. Bevorzugte Alkenylgruppen sind 1E-Alkenyl, 3E-Alkenyl und 4Z-Alkenyl. Bevorzugte Alkenyloxygruppen sind 2E-Alkenyloxy und 3Z-Alkenyloxy.

Die Erfindung wird durch die folgenden Beispiele weiter veranschaulicht. Im Zusammenhang mit Flüssigkristallphasen und Phasenübergängen bedeuten C eine kristalline Phase, $S_B$ eine smektische B Phase, N eine nematische Phase, N* eine cholesterische Phase und I die isotrope Phase. Die Helixganghöhe wird mit p bezeichnet und die Wellenlänge des selektiv reflektierten, zirkular polarisierten Lichts mit $\lambda_{max}$. Optische Antipoden besitzen jeweils "spiegelbildliche Eigenschaften", d.h. gleiche Schmelzpunkte etc., führen aber zu entgegengesetztem Helixdrehsinn und entgegengesetzter Zirkularpolarisation des reflektierten Lichts.

Beispiel 1

Eine Lösung von 0,8 g 4-[(E)-3-(trans-4-Pentylcyclohexyl)allyloxy]benzaldehyd und 1,0 g L(+)-Weinsäure-dimethylester in 50 ml Toluol wurde mit 2 Tropfen 10%iger (Vol.) Schwefelsäure versetzt. Das Gemisch wurde 2,5 Stunden zum Sieden erhitzt, wobei das entstandene Wasser gleichzeitig abdestilliert wurde. Dann wurden 4 Tropfen Triäthylamin zum Reaktionsgemisch zugegeben. Nach dem Erkalten wurde das Gemisch mit 20 ml 1N Natriumhydrogencarbonat-Lösung und zweimal mit je 20 ml Wasser gewaschen, über Natriumsulfat getrocknet, filtriert und eingeengt. Chromatographie des Rückstandes an Kieselgel mit Toluol/Aethylacetat (Vol. 1:1) und Umkristallisation aus Aethanol ergab reinen (4R,5R)--2-(4-[(E)-3-(trans-4-Pentylcyclohexyl)allyloxy]phenyl-1,3-dioxolan-4,5-dicarbonsäure-dimethylester;

In analoger Weise können folgende Verbindungen hergestellt werden:

(4R,5R)-2-(4-[(E)-3-(trans-4-Pentylcyclohexyl)allyloxy]phenyl)-1,3-dioxolan-4,5-dicarbonsäure-diäthylester;

(4R,5R)-2-(4-[(E)-3-(trans-4-Pentylcyclohexyl)allyloxy]phenyl)-1,3-dioxolan-4,5-dicarbonsäure-diisopropylester;

(4R,5R)-2-(4-[(E)-3-(trans-4-Pentylcyclohexyl)allyloxy]phenyl)-1,3-dioxolan-4,5-dicarbonsäure-diisobutylester;

(4R,5R)-2-(4-[3-(trans-4-Pentylcyclohexyl)-1-propyloxy]phenyl)-1,3-dioxolan-4,5-dicarbonsäure-dimethylester;

(4R,5R)-2-(4-[3-(trans-4-Pentylcyclohexyl)-1-propyloxy]phenyl)-1,3-dioxolan-4,5-dicarbonsäure-diäthylester;

(4R,5R)-2-(4-[3-(trans-4-Pentylcyclohexyl)-1-propyloxy]phenyl)-1,3-dioxolan-4,5-dicarbonsäure-diisopropylester;

(4R,5R)-2-(4-[3-(trans-4-Pentylcyclohexyl)-1-propyloxy]phenyl)-1,3-dioxolan-4,5-dicarbonsäure-dibutylester,
Smp. 42 °C;

(4R,5R)-2-(4-[4-(trans-4-Pentylcyclohexyl)-1-butyl]phenyl)-1,3-dioxolan-4,5-dicarbonsäure-dimethylester;

(4R,5R)-2-(4-[4-(trans-4-Pentylcyclohexyl)-1-butyl]phenyl)-1,3-dioxolan-4,5-dicarbonsäure-diäthylester;

(4R,5R)-2-(4-[4-(trans-4-Pentylcyclohexyl)-1-butyl]phenyl)-1,3-dioxolan-4,5-dicarbonsäure-diisopropylester;

(4R,5R)-2-(4-[4-(trans-4-Pentylcyclohexyl)-1-butyl]phenyl)-1,3-dioxolan-4,5-dicarbonsäure-dibutylester;

(4R,5R)-2-(4-[(E)-(trans-4-Pentylcyclohexyl)-3-butenyl]phenyl)-1,3-dioxolan-4,5-dicarbonsäure-dimethylester;

(4R,5R)-2-(4-[(E)-(trans-4-Pentylcyclohexyl)-3-butenyl]phenyl)-1,3-dioxolan-4,5-dicarbonsäure-diäthylester;

(4R,5R)-2-(4-[(E)-(trans-4-Pentylcyclohexyl)-3-butenyl]phenyl)-1,3-dioxolan-4,5-dicarbonsäure-diisopropylester;

(4R,5R)-2-(4-[(E)-(trans-4-Pentylcyclohexyl)-3-butenyl]phenyl)-1,3-dioxolan-4,5-dicarbonsäure-dibutylester;

(4R,5R)-2-(4-[(E)-3-(trans-4-Propylcyclohexyl)allyloxy]phenyl)-1,3-dioxolan-4,5-dicarbonsäure-dimethylester;

(4R,5R)-2-(4-[(E)-3-(trans-4-Propylcyclohexyl)allyloxy]phenyl)-1,3-dioxolan-4,5-dicarbonsäure-diäthylester;

(4R,5R)-2-(4-[(E)-3-(trans-4-Propylcyclohexyl)allyloxy]phenyl)-1,3-dioxolan-4,5-dicarbonsäure-diisopropylester;
(4R,5R)-2-(4-[(E)-3-(trans-4-Propylcyclohexyl)allyloxy]phenyl)-1,3-dioxolan-4,5-dicarbonsäure-diisobutylester;
(4R,5R)-2-(4-[3-(trans-4-Propylcyclohexyl)-1-propyloxy]phenyl)-1,3-dioxolan-4,5-dicarbonsäure-dimethylester;
(4R,5R)-2-(4-[3-(trans-4-Propylcyclohexyl)-1-propyloxy]phenyl)-1,3-dioxolan-4,5-dicarbonsäure-diäthylester;
(4R,5R)-2-(4-[3-(trans-4-Propylcyclohexyl)-1-propyloxy]phenyl)-1,3-dioxolan-4,5-dicarbonsäure-diisopropylester;
(4R,5R)-2-(4-[3-(trans-4-Propylcyclohexyl)-1-propyloxy]phenyl)-1,3-dioxolan-4,5-dicarbonsäure-dibutylester;
(4R,5R)-2-(4-[4-(trans-4-Propylcyclohexyl)-1-butyl]phenyl)-1,3-dioxolan-4,5-dicarbonsäure-dimethylester;
(4R,5R)-2-(4-[4-(trans-4-Propylcyclohexyl)-1-butyl]phenyl)-1,3-dioxolan-4,5-dicarbonsäure-diäthylester;
(4R,5R)-2-(4-[4-(trans-4-Propylcyclohexyl)-1-butyl]phenyl)-1,3-dioxolan-4,5-dicarbonsäure-diisopropylester;
(4R,5R)-2-(4-[4-(trans-4-Propylcyclohexyl)-1-butyl]phenyl)-1,3-dioxolan-4,5-dicarbonsäure-dibutylester;
(4R,5R)-2-(4-[(E)-(trans-4-Propylcyclohexyl)-3-butenyl]phenyl)-1,3-dioxolan-4,5-dicarbonsäure-dimethylester;
(4R,5R)-2-(4-[(E)-(trans-4-Propylcyclohexyl)-3-butenyl]phenyl)-1,3-dioxolan-4,5-dicarbonsäure-diäthylester;
(4R,5R)-2-(4-[(E)-(trans-4-Propylcyclohexyl)-3-butenyl]Phenyl)-1,3-dioxolan-4,5-dicarbonsäure-diisopropylester;
(4R,5R)-2-(4-[(E)-(trans-4-Propylcyclohexyl)-3-butenyl]phenyl)-1,3-dioxolan-4,5-dicarbonsäure-dibutylester;
4-[(4R,5R)-4,5-Dimethoxycarbonyl-1,3-dioxolan-2-yl]-4'-[(E)-3-(trans-4-pentylcyclohexyl)allyloxy]-biphenyl;
4-[(4R,5R)-4,5-Diäthoxycarbonyl-1,3-dioxolan-2-yl]-4'-[(E)-3-(trans-4-pentylcyclohexyl)allyloxy]biphenyl;
4-[(4R,5R)-4,5-Diisopropyloxycarbonyl-1,3-dioxolan-2-yl]-4'-[(E)-3-(trans-4-pentylcyclohexyl)allyloxy]-biphenyl;
4-[(4R,5R)-4,5-Diisobutyloxycarbonyl-1,3-dioxolan-2-yl]-4'-[(E)-3-(trans-4-pentylcyclohexyl)allyloxy]-biphenyl;
4-[(4R,5R)-4,5-Dimethoxycarbonyl-1,3-dioxolan-2-yl]-4'-[4-(trans-4-pentylcyclohexyl)-1-propyloxy]-diphenyl;
4-[(4R,5R)-4,5-Diäthoxycarbonyl-1,3-dioxolan-2-yl]-4'-[4-(trans-4-pentylcyclohexyl)-1-propyloxy]diphenyl;
4-[(4R,5R)-4,5-Diisopropyloxycarbonyl-1,3-dioxolan-2-yl]-4'-[4-(trans-4-pentylcyclohexyl)-1-propyloxy]-diphenyl;
4-[(4R,5R)-4,5-Dibutyloxycarbonyl-1,3-dioxolan-2-yl]-4'-[4-(trans-4-pentylcyclohexyl)-1-propyloxy]-diphenyl;
4-[(4R,5R)-4,5-Dimethoxycarbonyl-1,3-dioxolan-2-yl]-4'-[4-(trans-4-pentylcyclohexyl)-1-butyl]biphenyl;
4-[(4R,5R)-4,5-Diäthoxycarbonyl-1,3-dioxolan-2-yl]-4'-[4-(trans-4-pentylcyclohexyl)-1-butyl]biphenyl;
4-[(4R,5R)-4,5-Diisopropoxyoxycarbonyl-1,3-dioxolan-2-yl]-4'-[4-(trans-4-pentylcyclohexyl)-1-butyl]-biphenyl;
4-[(4R,5R)-4,5-Dibutyoxycarbonyl-1,3-dioxolan-2-yl]-4'-[4-(trans-4-pentylcyclohexyl)-1-butyl]biphenyl;
4-[(4R,5R)-4,5-Dimethoxycarbonyl-1,3-dioxolan-2-yl]-4'-[(E)-3-(trans-4-pentylcyclohexyl)butenyl]diphenyl;
4-[(4R,5R)-4,5-Diäthoxycarbonyl-1,3-dioxolan-2-yl]-4'-[(E)-3-(trans-4-pentylcyclohexyl)butenyl]diphenyl;
4-[(4R,5R)-4,5-Diisopropoxycarbonyl-1,3-dioxolan-2-yl]-4'-[(E)-3-(trans-4-pentylcyclohexyl)butenyl]-diphenyl;
4-[(4R,5R)-4,5-Dibutyloxycarbonyl-1,3-dioxolan-2-yl]-4'-[(E)-3-(trans-4-pentylcyclohexyl)butenyl]diphenyl;
4-[(4R,5R)-4,5-Dimethoxycarbonyl-1,3-dioxolan-2-yl]-4'-[(E)-3-(trans-4-propylcyclohexyl)allyloxy]-biphenyl;
4-[(4R,5R)-4,5-Diäthoxycarbonyl-1,3-dioxolan-2-yl]-4'-[(E)-3-(trans-4-propylcyclohexyl)allyloxy]biphenyl;
4-[(4R,5R)-4,5-Diisopropyloxycarbonyl-1,3-dioxolan-2-yl]-4'-[(E)-3-(trans-4-propylcyclohexyl)allyloxy]-biphenyl;
4-[(4R,5R)-4,5-Diisobutyloxycarbonyl-1,3-dioxolan-2-yl]-4'-[(E)-3-(trans-4-propylcyclohexyl)allyloxy]-biphenyl;
4-[(4R,5R)-4,5-Dimethoxycarbonyl-1,3-dioxolan-2-yl]-4'-[4-(trans-4-propylcyclohexyl)-1-propyloxy]-diphenyl;

4-[(4R,5R)-4,5-Diäthoxycarbonyl-1,3-dioxolan-2-yl]-4'-[4-(trans-4-propylcyclohexyl)-1-propyloxy]diphenyl;
4-[(4R,5R)-4,5-Diisopropyloxycarbonyl-1,3-dioxolan-2-yl]-4'-[4-(trans-4-propylcyclohexyl)-1-propyloxy]-diphenyl;
4-[(4R,5R)-4,5-Dibutyloxycarbonyl-1,3-dioxolan-2-yl]-4'-[4-(trans-4-propylcyclohexyl)-1-propyloxy]-diphenyl;
4-[(4R,5R)-4,5-Dimethoxycarbonyl-1,3-dioxolan-2-yl]-4'-[4-(trans-4-propylcyclohexyl)-1-butyl]biphenyl;
4-[(4R,5R)-4,5-Diäthoxycarbonyl-1,3-dioxolan-2-yl]-4'-[4-(trans-4-propylcyclohexyl)-1-butyl]biphenyl;
4-[(4R,5R)-4,5-Diisopropoxyoxycarbonyl-1,3-dioxolan-2-yl]-4'-[4-(trans-4-propylcyclohexyl)-1-butyl]-biphenyl;
4-[(4R,5R)-4,5-Dibutyoxycarbonyl-1,3-dioxolan-2-yl]-4'-[4-(trans-4-propylcyclohexyl)-1-butyl]biphenyl;
4-[(4R,5R)-4,5-Dimethoxycarbonyl-1,3-dioxolan-2-yl]-4'-[(E)-3-(trans-4-propylcyclohexyl)butenyl]-diphenyl;
4-[(4R,5R)-4,5-Diäthoxycarbonyl-1,3-dioxolan-2-yl]-4'-[(E)-3-(trans-4-propylcyclohexyl)butenyl]diphenyl;
4-[(4R,5R)-4,5-Diisopropoxycarbonyl-1,3-dioxolan-2-yl]-4'-[(E)-3-(trans-4-propylcyclohexyl)butenyl]-diphenyl;
4-[(4R,5R)-4,5-Dibutyloxycarbonyl-1,3-dioxolan-2-yl]-4'-[(E)-3-(trans-4-propylcyclohexyl)butenyl]diphenyl.

Beispiel 2

Eine Lösung von 1,0 g Terephthaldehyd, 5,8 g (R,R)-2,3-bis-(trimethylsiloxy)bernsteinsäure-dimethylester in 25 ml Aethylenchlorid wurde bei 0 °C und unter Stickstoffbegasung mit 0,14 ml Bortrifluorid-äthylätherat versetzt, 15 Minuten gerührt, mit 0,06 ml Trifluormethansulfonsäure versetzt, noch 30 Minuten bei 0 °C, dann über Nacht bei Raumtemperatur gerührt. Das Gemisch wurde auf 0 °C abgekühlt, tropfenweise mit 25 ml konzentrierter Natriumcarbonat-Lösung versetzt und noch 15 Minuten bei Raumtemperatur weitergerührt. Die organische Phase wurde abgetrennt und die wässrige Phase zweimal mit je 25 ml Dichlormethan nachextrahiert. Die vereinigten organischen Phasen wurden zweimal mit je 100 ml Wasser gewaschen, über Magnesiumsulfat getrocknet, filtriert und anschliessend eingeengt. Der Rückstand wurde an Kieselgel mit Hexan/Aethylacetat (Vol. 1:1) chromatographisch gereinigt. Umkristallisation aus einem Hexan/Aethylacetat-Gemisch (Vol. 1:1) ergab reinen 2,2'-(4-Phenylen)-bis-(4R,5R)-1,3-dioxolan-4,5-dicarbonsäure-dimethylester mit Smp. (C-I) 88 °C;

Der als Ausgangsmaterial verwendete (R,R)-2,3-bis-(Trimethylsiloxy)bernsteinsäure-dimethylester wurde wie folgt hergestellt:

Eine Lösung von 20 g L(+)-Weinsäure-dimethylester und 35 ml Triäthylamin in 200 ml Toluol wurde bei -5 °C und unter Stickstoffbegasung mit 32 g Trimethylchlorsilan tropfenweise versetzt. Es bildete sich ein weisses Reaktionsgemisch, das noch 90 Minuten bei Raumtemperatur gerührt wurde. Das Gemisch wurde mit 100 ml gesättigter Natriumbicarbonat-Lösung versetzt und dann dreimal mit je 50 ml Aethylacetat extrahiert. Die vereinigten organischen Phasen wurden zweimal mit je 100 ml gesättigter Natriumbicarbonat-Lösung gewaschen, über Magnesiumsulfat getrocknet, filtriert und dann eingeengt. Destillation des Rückstandes ergab 36 g (R,R)-2,3-bis-(Trimethylsiloxy)bernsteinsäure-dimethylester, Sdp. 123-125 °C/0,07 mmHg.

In analoger Weise können folgende Verbindungen hergestellt werden:

2,2'-(4-Phenylen)-bis-(4R,5R)-1,3-dioxolan-4,5-dicarbonsäure-diäthylester, Smp. 75 °C;
2,2'-(4-Phenylen)-bis-(4R,5R)-1,3-dioxolan-4,5-dicarbonsäure-diisopropylester, Smp. 78 °C;
2,2'-(4-Phenylen)-bis-(4R,5R)-1,3-dioxolan-4,5-dicarbonsäure-dibutylester, Smp. 41 °C;
2,2'-(4,4'-Biphenylyl)-bis-[(4R,5R)-1,3-dioxolan-4,5-dicarbonsäure-dimethylester, Smp. 133 °C;
2,2'-(4,4'-Biphenylyl)-bis-[(4R,5R)-1,3-dioxolan-4,5-dicarbonsäure-diäthylester, Smp. 79 °C;
2,2'-(4,4'-Biphenylyl)-bis-[(4R,5R)-1,3-dioxolan-4,5-dicarbonsäure-diisopropylester, Smp. 97 °C;
2,2'-(4,4'-Biphenylyl)-bis-[(4R,5R)-1,3-dioxalan-4,5-dicarbonsäure-dibutylester, Smp. 67 °C.

Beispiel 3

Zur Messung der induzierten Ganghöhe (Pitch) und deren Temperaturabhängigkeit in Flüssigkristallmaterialien wurde die folgende Flüssigkristall-Grundmischung BM-1 verwendet:

5,36 Gew.% 4'-Aethyl-4-cyanobiphenyl,
3,18 Gew.% 4'-Propyl-4-cyanobiphenyl,
6,08 Gew.% 4'-Butyl-4-cyanobiphenyl,
6,53 Gew.% 4-(trans-4-Propylcyclohexyl)benzonitril,

14,67 Gew.% 4-(trans-4-Pentylcyclohexyl)benzonitril,
5,21 Gew.% 4-Aethyl-1-(trans-4-propylcyclohexyl)benzol,
16,54 Gew.% 4-Aethoxy-1-[2-(trans-4-propylcyclohexyl)-äthyl]benzol,
5,60 Gew.% 4"-Pentyl-4-cyano-p-terphenyl,
5,71 Gew.% 4'-(trans-4-Pentylcyclohexyl)-4-cyanobiphenyl,
15,95 Gew.% 1-[2-(trans-4-Butylcyclohexyl)äthyl]-4-(trans-4-pentylcyclohexyl)benzol,
4,74 Gew.% 4-[2-(trans-4-Butylcyclohexyl)äthyl]-4'-(trans-4-pentylcyclohexyl)biphenyl,
7,59 Gew.% 4-[2-(trans-4-Butylcyclohexyl)äthyl]-4'-(trans-4-pentylcyclohexyl)-1,1'-äthylendibenzol,
2,84 Gew.% trans-4-[2-(trans-4-Propylcyclohexyl)äthyl]-cyclohexancarbonsäure-4-cyanophenylester;
Smp. <-30 °C, Klp. (N-I) 90 °C; $\Delta\epsilon$ = 8,5, $\Delta n$ = 0,139 und $\eta$ = 22 mPa•s gemessen bei 22 °C.

Flüssigkristall-Grundmischung BM-1 wurde jeweils mit einem der folgenden optisch aktiven Dotierstoffe versetzt:

D-1 = 2,2'-(4-Phenyl)-bis-(4R,5R)-1,3-dioxolan-4,5-dicarbonsäure-dimethylester,
D-2 = 2,2'-(4,4'-biphenyl)-bis-(4R,5R)-1,3-dioxolan-4,5-dicarbonsäure-diisopropylester,

Für die chiral dotierten Gemische wurden die in Tabelle 1 zusammengestellten Ergebnisse erhalten, wobei A, B und C die Parameter der Reihenentwicklung

$$\frac{1}{pc} = A + BT_1 + CT_1^2$$

bezeichnen und p, c und $T_1$ die folgenden Bedeutungen haben:

$T_1$ = T-22 °C
T = Temperatur in °C
p = Ganghöhe in µm (ein positiver Wert bedeutet rechtsdrehende Helixstruktur und ein negativer Wert bedeutet linksdrehende Helixstruktur)
c = Konzentration des optisch aktiven Dotierstoffes in Gew.-%.

Tabelle 1

| Mischung | Dotierstoff | A $[10^{-2} \cdot \mu m^{-1} \cdot Gew.\text{-}\%^{-1}]$ | B $[10^{-4} \cdot \mu m^{-1} \cdot Gew.\text{-}\%^{-1} \cdot °C^{-1}]$ | C $[10^{-6} \cdot \mu m^{-1} \cdot Gew.\text{-}\%^{-1} \cdot °C^{-2}]$ | p•c (bei 22°C) [µm•Gew.-%] |
|---|---|---|---|---|---|
| M-1 | 1,0 Gew.-% D-1 | -25,50 | - 15,92 | 0,77 | -3,92 |
| M-2 | 1,0 Gew.-% D-2 | -26,86 | 8,82 | -2,00 | -3,72 |

## Patentansprüche

1. Optisch aktive Verbindungen der allgemeinen Formel

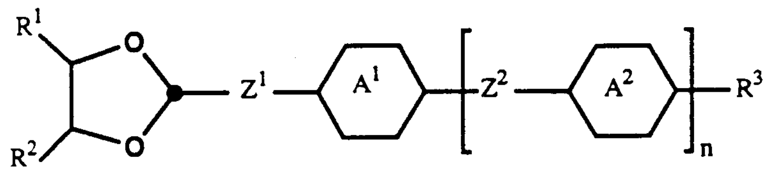

I

worin n für die Zahl 0 oder 1 steht; $R^3$ eine Gruppe der allgemeinen Formel

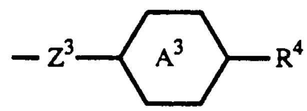

II

oder

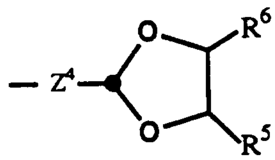

III

bezeichnet; die Ringe $A^1$, $A^2$ und $A^3$ unabhängig voneinander unsubstituiertes oder mit Halogen, Cyano und/oder Methyl substituiertes 1,4-Phenylen, in welchem gegebenenfalls 1 oder 2 CH-Gruppen durch Stickstoff ersetzt sind, trans-1,4-Cyclohexylen, trans-1,3-Dioxan-2,5-diyl oder Naphthalin-2,6-diyl darstellen und $A^2$ zusätzlich 4,4'-Biphenylen darstellt; $Z^1$ und $Z^4$ unabhängig voneinander eine einfache Kovalenzbindung oder $-CH_2CH_2-$ bedeuten; $Z^2$ und $Z^3$ unabhängig voneinander eine einfache Kovalenzbindung, $-CH_2CH_2-$, -COO-, -OOC-, $-CH_2O-$, $-OCH_2-$, -C≡C-, $-(CH_2)_4-$, $-(CH_2)_3O-$, $-O(CH_2)_3-$ oder die trans-Form von $-CH=CH-CH_2CH_2-$, $-CH_2CH_2-CH=CH-$, $-CH=CH-CH_2O-$ oder $-OCH_2-CH=CH-$ bezeichnen, mit der Massgabe, dass, wenn $R^3$ eine Gruppe der Formel II bedeutet, mindestens eine der Gruppen $Z^2$ und $Z^3$ $-(CH_2)_4-$, $-(CH_2)_3-O-$, $-O-(CH_2)_3-$ oder die trans-Form von $-CH=CH-CH_2-CH_2-$, $-CH_2-CH_2-CH=CH-$, $-CH=CH-CH_2-O-$ oder $-O-CH_2-CH=CH-$ bedeutet; $R^1$, $R^5$ und $R^6$ Wasserstoff, Alkyl, Phenyl oder Alkoxycarbonyl bedeuten; $R^2$ Alkyl, Phenyl oder Alkoxycarbonyl bedeutet; $R^4$ Halogen, Cyano, Trifluormethyl, Trifluormethoxy oder Alkyl bezeichnet, in welchem gegebenenfalls eine $-CH_2-CH_2-$ durch -CH=CH- ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist; X Halogen, Cyano oder Methyl bedeutet; und der in Formel I dargestellte Dioxolan-Ring in optisch aktiver Form vorliegt.

2. Optisch aktive Verbindungen nach Anspruch 1 mit der relativen Konfiguration der allgemeinen Formel

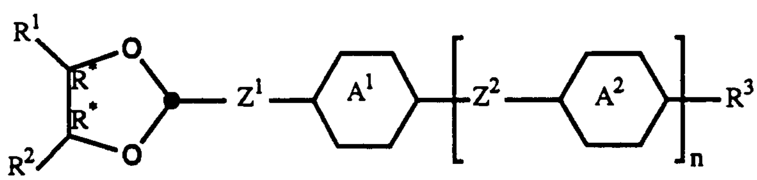

IA

worin $R^3$ eine Gruppe der allgemeinen Formel

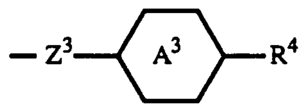

II

oder

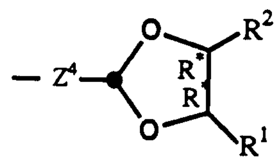

IIIA

bezeichnet; n, $A^1$, $A^2$, $A^3$, $Z^1$, $Z^2$, $Z^3$, $Z^4$, $R^1$, $R^2$ und $R^4$ die in Anspruch 1 gegebenen Bedeutungen haben, mit der Massgabe, dass, wenn $R^3$ eine Gruppe der Formel II bedeutet, mindestens eine der Gruppen $Z^2$ und $Z^3$ $-(CH_2)_4-$, $-(CH_2)_3-O-$, $-O-(CH_2)_3-$ oder die trans-Form von $-CH=CH-CH_2-CH_2-$, $-CH_2-CH_2-CH=CH-$, $-CH=CH-CH_2-O-$ oder $-O-CH_2-CH=CH-$ bedeutet; und mit R* bezeichnete, asymmetrische Kohlenstoffatome alle in der R-Konfiguration oder alle in der S-Konfiguration vorliegen.

**3.** Optisch aktive Verbindungen nach Anspruch 1 mit der allgemeinen Formel

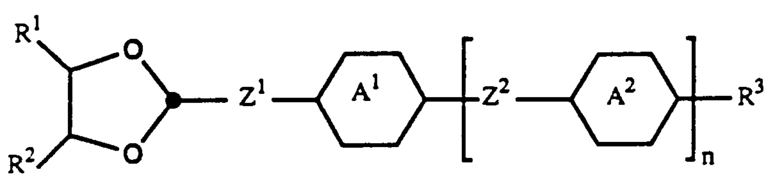

I

worin $R^3$ eine Gruppe der allgemeinen Formel

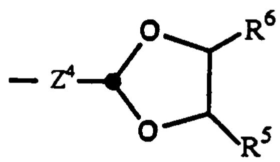

III

bezeichnet; n, $R^1$, $R^2$, $R^5$, $R^6$, $Z^1$, $Z^2$, $Z^4$, die Ringe $A^1$ und $A^2$ die in Anspruch 1 angegebenen Bedeutungen haben.

**4.** Optisch aktive Verbindungen nach einem der Ansprüche 1 bis 3 mit der allgemeinen Formel

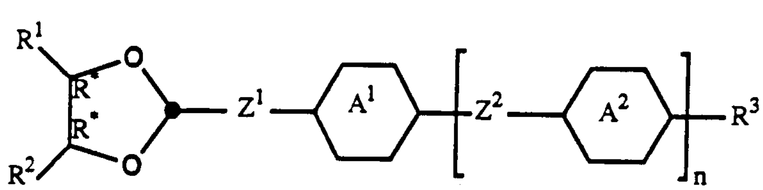

IA

worin $R^3$ eine Gruppe der allgemeinen Formel

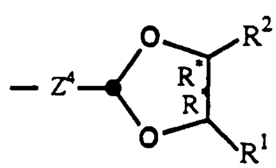

IIIA

bezeichnet; $R^1$, $R^2$, $Z^1$, $Z^2$, $Z^4$, die Ringe $A^1$ und $A^2$ die in Anspruch 2 angegebenen Bedeutungen haben.

**5.** Optisch aktive Verbindungen nach Anspruch 1 oder 2, dadurch gekennzeichnet, dass einer der Ringe $A^1$, $A^2$ und $A^3$ unsubstituiertes oder mit Halogen, Cyano und/oder Methyl substituiertes 1,4-Phenylen oder Pyridin-2,5-diyl, Pyrimidin-2,5-diyl, trans-1,4-Cyclohexylen, trans-1,3-Dioxan-2,5-diyloder Naphthalin-2,6-diyl darstellt und $A^2$ zusätzlich 4,4'-Biphenylen bedeutet, die andern der Ringe $A^1$, $A^2$ und $A^3$ unabhängig voneinander unsubstituiertes oder mit Halogen, Cyano und/oder Methyl substituiertes 1,4-Phenylen oder trans-1,4-Cyclohexylen darstellen.

**6.** Optisch aktive Verbindungen nach Anspruch 5, dadurch gekennzeichnet, dass $A^1$, $A^2$ und $A^3$ unabhängig voneinander unsubstituiertes oder mit Halogen, Cyano und/oder Methyl substituiertes 1,4-Phenylen oder trans-1,4-Cyclohexylen darstellen oder eine der Gruppen $A^1$, $A^2$ und $A^3$ auch Pyrimidin-2,5-diyl darstellt, eine der Gruppen $Z^2$ und $Z^3$ $-(CH_2)_4-$, $-(CH_2)_3-O-$, $-O-(CH_2)_3-$ oder die trans-Form von $-CH=CH-CH_2-CH_2-$, $-CH_2-CH_2-CH=CH-$, $-CH=CH-CH_2-O-$ oder $-O-CH_2-CH=CH-$ bezeichnet, und die andere der Gruppen $Z^2$ und $Z^3$ eine einfache Kovalenzbindung bezeichnet.

**7.** Optisch aktive Verbindungen nach einem der Ansprüche 1 bis 6, gekennzeichnet durch die allgemeinen Formeln

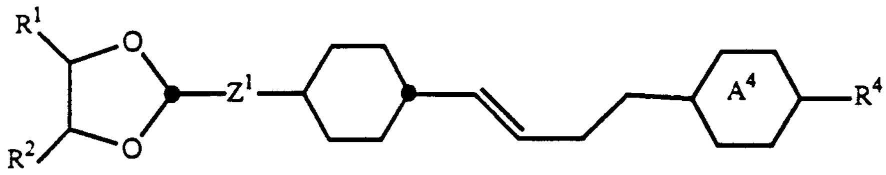

I-5

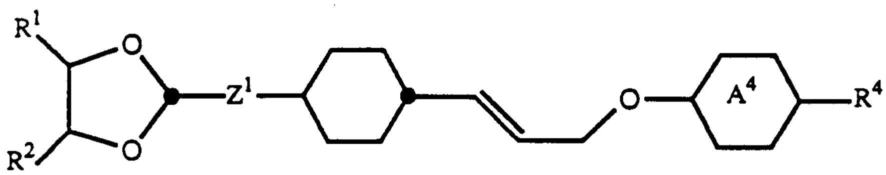

I-6

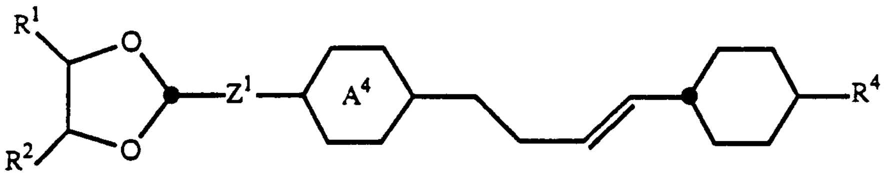

I-7

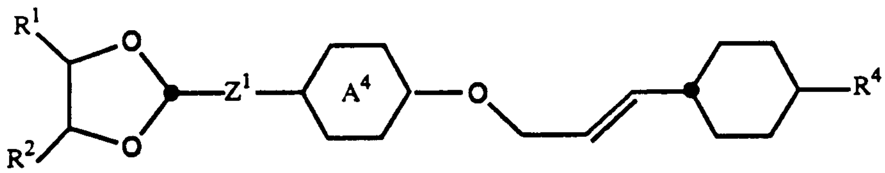

I-8

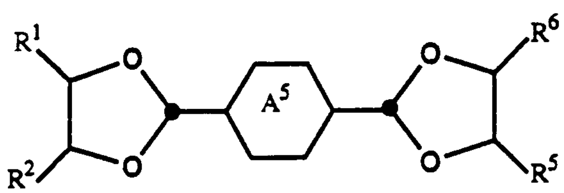

I-9

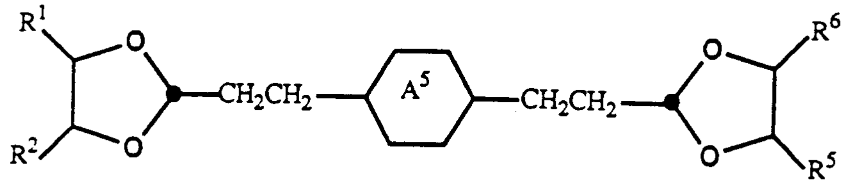

I-10

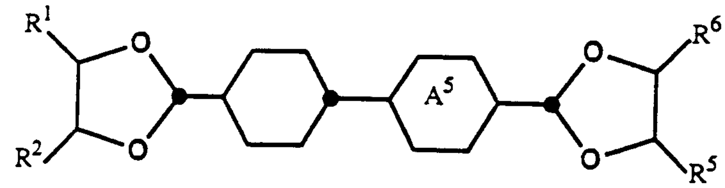

I-11

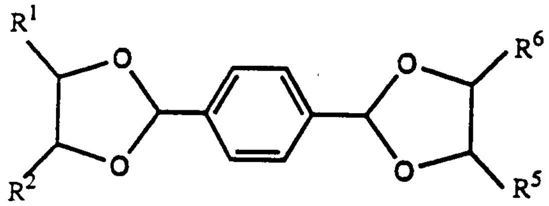

I-12

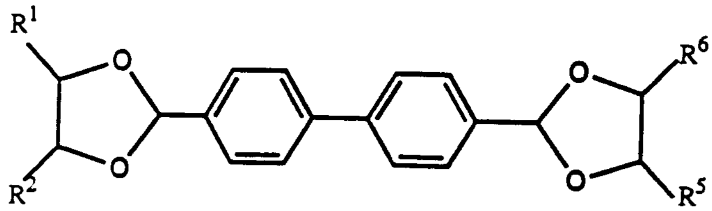

I-13

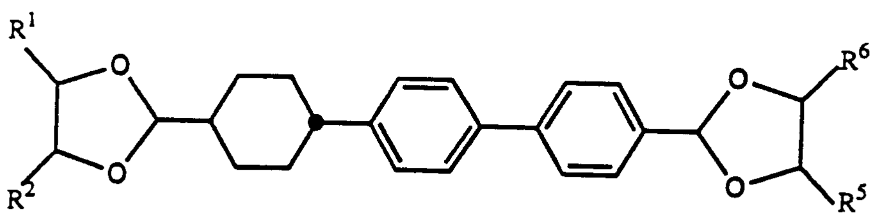

I-14

worin $R^1$, $R^2$ und $R^4$ die in Anspruch 1 gegebenen Bedeutungen haben; $R^5$ und $R^6$ die in Anspruch 1 gegebenen Bedeutungen haben, vorzugsweise $R^5$ die gleiche Bedeutung und am benachbarten C-Atom die gleiche Konfiguration wie $R^1$ besitzt und $R^6$ die gleiche Bedeutung und am benachbarten C-Atom die gleiche Konfiguration wie $R^2$ besitzt; Ringe $A^4$ und $A^5$ unsubstituiertes oder mit Halogen, Cyano und/oder Methyl substituiertes 1,4-Phenylen oder trans-1,4-Cyclohexylen darstellen und $A^5$ zusätzlich 4,4'-Biphenylen bedeutet.

**8.** Optisch aktive Verbindungen nach einem der Ansprüche 1, 2 und 5 bis 7, dadurch gekennzeichnet, dass $R^4$ Halogen, Cyano, Trifluormethyl, Trifluormethoxy, Alkyl, Alkenyl, Alkoxy, Alkenyloxy, 1-(Alkoxycarbonyl)äthoxy oder 1-(Alkoxycarbonyl)äthoxycarbonyl bezeichnet.

**9.** Optisch aktive Verbindungen nach einem der Ansprüche 1, 2 und 5 bis 8, dadurch gekennzeichnet, dass $R^4$ 1 bis 12, vorzugsweise 1 bis 7 Kohlenstoffatome besitzt.

**10.** Optisch aktive Verbindungen nach einem der Ansprüche 1 bis 9, dadurch gekennzeichnet, dass $R^1$ und $R^2$ $C_2$-$C_7$-Alkoxycarbonyl bezeichnen oder $R^1$ und $R^2$ Methyl bezeichnen oder $R^1$ und $R^2$ Phenyl bezeichnen oder $R^1$ Wasserstoff und $R^2$ Methyl oder Phenyl bezeichnen, $R^5$ die Bedeutung von $R^1$ hat, $R^6$ die Bedeutung von $R^2$ hat und die asymmetrischen Kohlenstoffatome die in Anspruch 2 angegebene relative Konfiguration aufweisen.

**11.** Flüssigkristallines Gemisch enthaltend ein flüssigkristallines Trägermaterial und eine oder mehrere optisch aktive Verbindungen gemäss Ansprüchen 1 bis 10.

**12.** Verwendung flüssigkristalliner Gemische gemäss Anspruch 9 für optische oder elektro-optische Zwekke.

**Claims**

**1.** Optically active compounds of the general formula

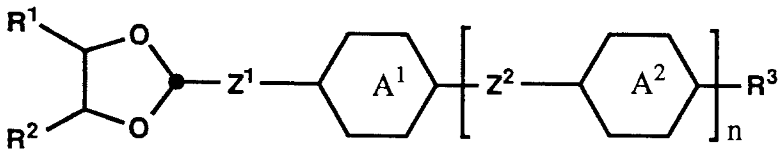

I

in which n represents the number 0 or 1; $R^3$ denotes a group of the general formula

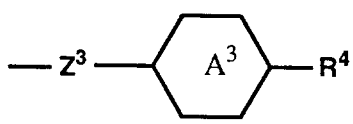

II

or

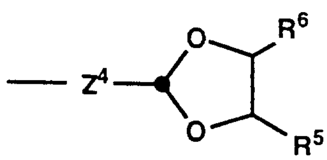

III

rings $A^1$, $A^2$ and $A^3$ each independently represent 1,4-phenylene, which is unsubstituted or substituted by halogen, cyano and/or methyl and in which 1 or 2 CH groups may be replaced by nitrogen, trans-1,4-cyclohexylene, trans-1,3-dioxane-2,5-diylor naphthalene-2,6-diyl, and ring $A^2$ additionally represents 1,4-biphenylene; $Z^1$ and $Z^4$ each independently denote a single covalent bond or $-CH_2CH_2-$; $Z^2$ and $Z^3$ each independently denote a single covalent bond, $-CH_2CH_2-$, -COO-, -OOC-, $-CH_2O-$, $-OCH_2-$, -C≡C-, $-(CH_2)_4-$, $-(CH_2)_3O-$, $-O(CH_2)_3-$ or the trans form of $-CH=CH-CH_2CH_2-$, $-CH_2CH_2CH=CH-$, $-CH=CH-CH_2-O-$ or $-O-CH_2-CH=CH-$; with the proviso that, when $R^3$ denotes a group of formula II, at least one of groups $Z^2$ and $Z^3$ denotes $-(CH_2)_4-$, $-(CH_2)_3-O-$, $-O-(CH_2)_3-$ or the trans form of $-CH=CH-CH_2-CH_2-$, $-CH_2-CH_2-CH=CH-$, $-CH=CH-CH_2-O-$ or $-O-CH_2-CH=CH-$; $R^1$, $R^5$ and $R^6$ denote hydrogen, alkyl, phenyl or alkoxycarbonyl; $R^2$ denotes alkyl, phenyl or alkoxycarbonyl; $R^4$ denotes halogen, cyano, trifluoromethyl, trifluoromethoxy or alkyl in which one $-CH_2-CH_2-$ may be replaced by -CH=CH- and/or one or two non-adjacent methylene groups may be replaced by -O-, -COO- and/or -OOC- and/or one methylene group may be replaced by -CHX-; X denotes halogen, cyano or methyl; and the dioxolane ring shown in formula I is in optically active form.

**2.** Optically active compounds according to claim 1, having the relative configuration of the general formula

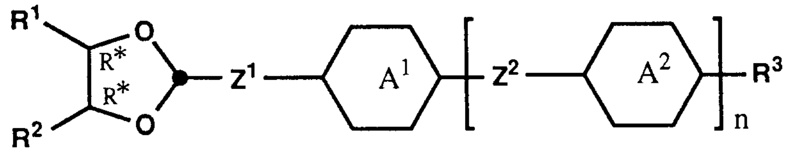

IA

in which $R^3$ denotes a group of the general formula

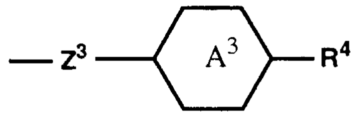

II

or

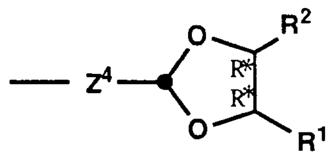

IIIA

n, $A^1$, $A^2$, $A^3$, $Z^1$, $Z^2$, $Z^3$, $Z^4$, $R^1$, $R^2$ and $R^4$ have the significances given in claim 1, with the proviso that, when $R^3$ denotes a group of formula II, at least one of groups $Z^2$ and $Z^3$ denotes $-(CH_2)_4-$, $-(CH_2)_3-O-$, $-O-(CH_2)_3-$ or the trans form of $-CH=CH-CH_2-CH_2-$, $-CH_2-CH_2-CH=CH-$, $-CH=CH-CH_2-O-$ or $-O-CH_2-CH=CH-$; and the asymmetric carbon atoms labeled R* are all in the R-configuration or are all in the S-configuration.

**3.** Optically active compounds according to claim 1 of the general formula

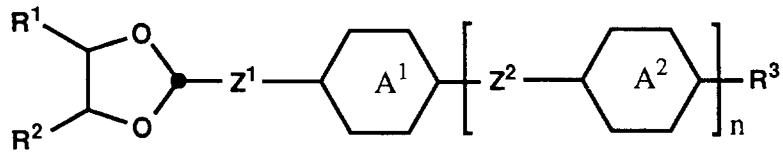

I

in which $R^3$ denotes a group of the general formula

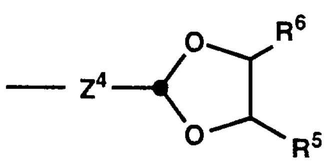

III

n, $R^1$, $R^2$, $R^5$, $R^6$, $Z^1$, $Z^2$, $Z^4$, rings $A^1$ and $A^2$ have the significances given in claim 1.

**4.** Optically active compounds according to any one of claims 1 to 3 of the general formula

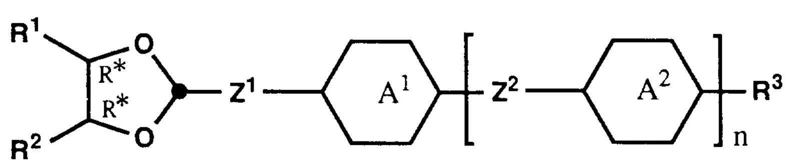

IA

in which $R^3$ denotes a group of the general formula

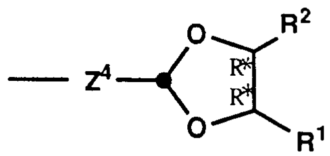

IIIA

$R^1$, $R^2$, $Z^1$, $Z^2$, $Z^4$, rings $A^1$ and $A^2$ have the significances given in claim 2.

**5.** Optically active compounds according to claim 1 or 2, characterized in that rings $A^1$, $A^2$ and $A^3$ denotes 1,4-phenylene, which is unsubstituted or substituted by halogen, cyano and/or methyl, pyridine-2,5-diyl, pyrimidine-2,5-diyl, trans-1,4-cyclohexylene, trans-1,3-dioxane-2,5-diyl or naphthalene-2,6-diyl and $A^2$ additionally denotes 4,4'-biphenylene, and the others of rings $A^1$, $A^2$ and $A^3$ each independently represent 1,4-phenylene, which is unsubstituted or substituted by halogen, cyano and/or methyl, or trans-1,4-cyclohexylene.

**6.** Optically active compounds according to claim 5, characterized in that rings $A^1$, $A^2$ and $A^3$ each independently represent 1,4-phenylene, which is unsubstituted or substituted by halogen, cyano and/or methyl, or trans-1,4-cyclohexylene or one of groups $A^1$, $A^2$ and $A^3$ also represents pyrimidine-2,5-diyl, one of groups $Z^2$ and $Z^3$ denotes $-(CH_2)_4-$, $-(CH_2)_3-O-$, $-O(CH_2)_3-$ or the transform of $-CH=CH-CH_2-CH_2-$, $-CH_2-CH_2-CH=CH-$, $-CH=CH-CH_2-O-$ or $-O-CH_2-CH=CH-$ and the other of groups $Z^2$ and $Z^3$ denotes a single covalent bond.

**7.** Optically active compounds according to any one of claims 1 to 6, characterized by the general formulae

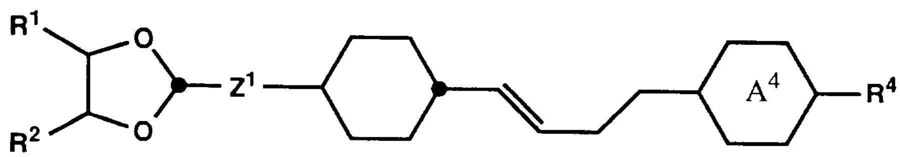

I-5

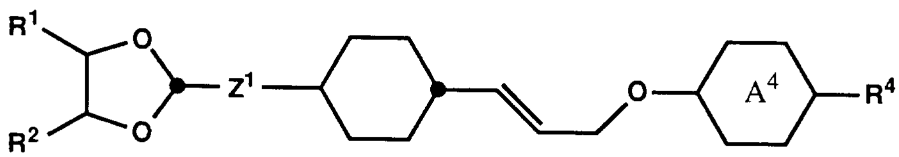

I-6

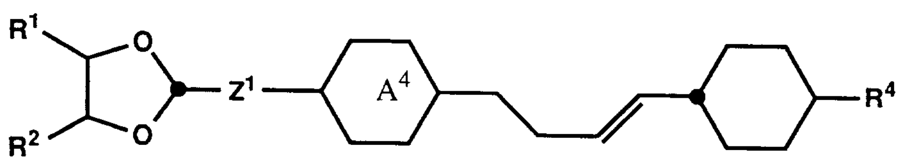

I-7

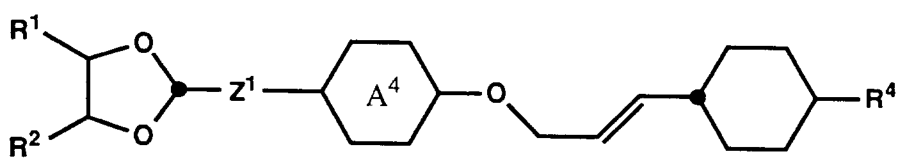

I-8

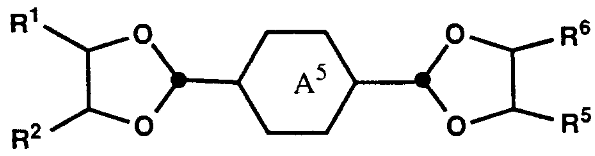

I-9

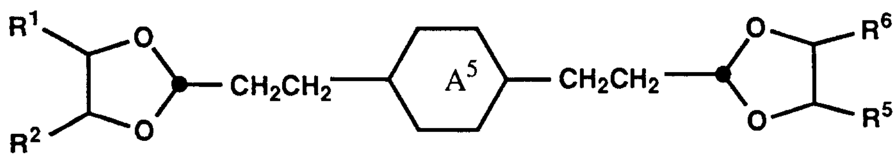

I-10

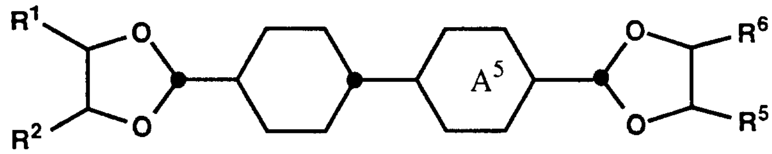

I-11

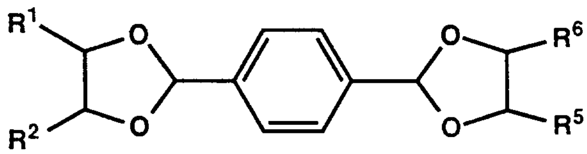

I-12

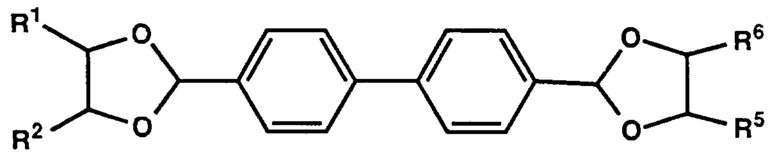

I-13

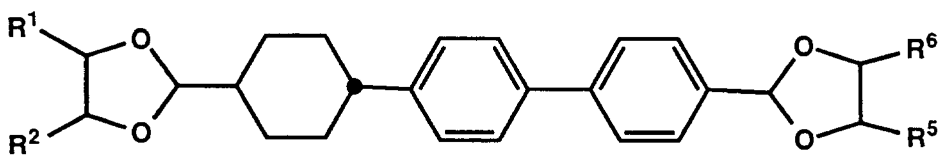

I-14

in which $R^1$, $R^2$ and $R^4$ have the significances given in claim 1; $R^5$ and $R^6$ have the significances given in claim 1, with $R^5$ preferably having the same meaning and the same configuration at the adjacent C atom as $R^1$ and $R^6$ preferably having the same meaning and the same configuration at the adjacent C atom as $R^2$; rings $A^4$ and $A^5$ represent 1,4-phenylene, which is unsubstituted or substituted by halogen, cyano and/or methyl, or trans-1,4-cyclohexylene and $A^5$ additionally denotes 4,4'-biphenylene.

**8.** Optically active compounds according to any one of claims 1, 2 and 5 to 7, characterized in that $R^4$ denotes halogen, cyano, trifluoromethyl, trifluoromethoxy, alkyl, alkenyl, alkoxy, alkenyloxy, 1-(alkoxycarbonyl)ethoxy or 1-(alkoxycarbonyl)-ethoxycarbonyl.

**9.** Optically active compounds according to any one of claims 1, 2 and 5 to 8, characterized in that $R^4$ has 1 to 12, preferably 1 to 7, carbon atoms.

**10.** Optically active compounds according to any one of claims 1 to 9, characterized in that $R^1$ and $R^2$ denote $C_2$-$C_7$-alkoxycarbonyl or $R^1$ and $R^2$ denote methyl or $R^1$ and $R^2$ denote phenyl or $R^1$ denotes hydrogen and $R^2$ denotes methyl or phenyl, $R^5$ has the meaning of $R^1$, $R^6$ has the meaning of $R^2$ and the asymmetric carbon atoms have the relative configuration indicated in claim 2.

**11.** Liquid crystalline mixture containing a liquid crystalline carrier material and one or more optically active compounds according to claims 1 to 10.

**12.** Use of liquid crystalline mixtures according to claim 11 for optical and electro-optical purposes.

**Revendications**

**1.** Composés possédant l'activité optique et répondant à la formule générale

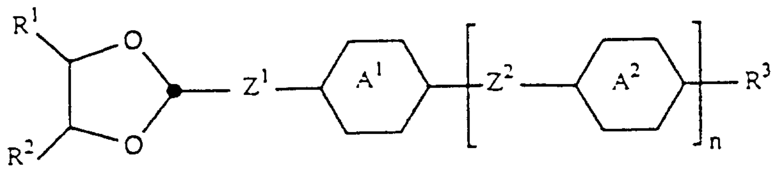

I

dans laquelle n est égal à 0 ou 1 ; $R^3$ représente un groupe de formule générale

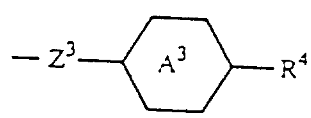

II

ou

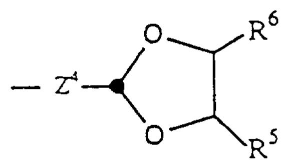

III

les cycles $A^1$, $A^2$ et $A^3$ sont, indépendamment les uns des autres, des cycles 1,4-phénylène non substitués ou substitués par des halogènes, des groupes cyano et/ou méthyle et dans lesquels, le cas échéant, un ou deux groupes CH sont remplacés par de l'azote, des cycles trans-1,4-cyclohexylène, trans-1,3-dioxanne-2,5-diyle ou naphtalène-2,6-diyle, $A^2$ pouvant en outre consister en un cycle 4,4'-biphénylène ; $Z^1$ et $Z^4$ représentent chacun, indépendamment l'un de l'autre, une liaison covalente simple ou $-CH_2CH_2-$ ; $Z^2$ et $Z^3$ représentent chacun, indépendamment l'un de l'autre, une liaison covalente simple, $-CH_2CH_2-$, -COO-, -OOC-, $-CH_2O-$, $-OCH_2$, -C≡C-, $-(CH_2)_4-$, $-(CH_2)_3O-$, $-O(CH_2)_3$ ou la forme trans de $-CH=CH-CH_2CH_2-$, $-CH_2CH_2-CH=CH-$, $-CH=CH-CH_2O-$ ou $-OCH_2-CH=CH-$, sous réserve que lorsque $R^3$ représente un groupe de formule II, au moins un des symboles $Z^2$ et $Z^3$ représente $-(CH_2)_4-$, $-(CH_2)_3-O-$, $-O-(CH_2)_3-$ ou la forme trans de $-CH=CH-CH_2-CH_2-$, $-CH_2-CH_2-CH=CH-$, $-CH=CH-CH_2-O-$ou $-O-CH_2-CH=CH-$ ; $R^1$, $R^5$ et $R^6$ représentent l'hydrogène, des groupes alkyle, phényle ou alcoxycarbonyle ; $R^2$ représente un groupe alkyle, phényle ou alcoxycarbonyle ; $R^4$ représente un halogène, un groupe cyano, trifluorométhyle, trifluorométhoxy ou alkyle dans lequel, le cas échéant, un groupe $-CH_2-CH_2-$ est remplacé par -CH=CH- et/ou, le cas échéant, un ou deux groupes méthylène non voisins sont remplacés par -O-, -COO- et/ou -OOC- et/ou, le cas échéant, un groupe méthylène est remplacé par -CHX- ; X représente un halogène, un groupe cyano ou méthyle ;

et le cycle dioxolanne de la formule I est sous une forme présentant l'activité optique.

**2.** Composés, possédant l'activité optique, selon revendication 1, à la configuration relative de la formule générale

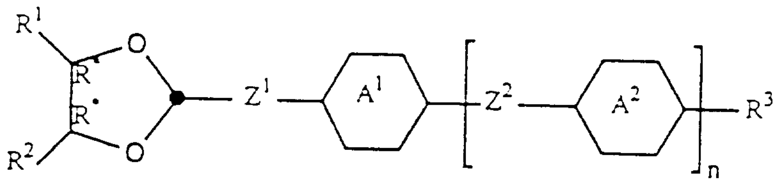

IA

dans laquelle $R^3$ représente un groupe de formule générale

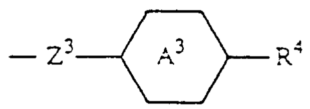

II

ou

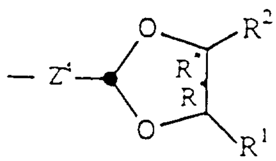

IIIA

n, $A^1$, $A^2$, $A^3$, $Z^1$, $Z^2$, $Z^3$, $Z^4$, $R^2$ et $R^4$ ont les significations indiquées dans la revendication 1, sous réserve que lorsque $R^3$ représente un groupe de formule II, au moins un des symboles $Z^2$ et $Z^3$ représente $-(CH_2)_4-$,$-(CH_2)_3-O-$, $-O-(CH_2)_3-$ ou la forme trans de $-CH=CH-CH_2-CH_2-$, $-CH_2-CH_2-CH=CH-$, $-CH=CH-CH_2-O-$ ou $-O-CH_2-CH=CH-$ ; et les atomes de carbone asymétriques signalés par le symbole R* sont tous en configuration R ou tous en configuration S.

**3.** Composés possédant l'activité optique selon revendication 1, de formule générale

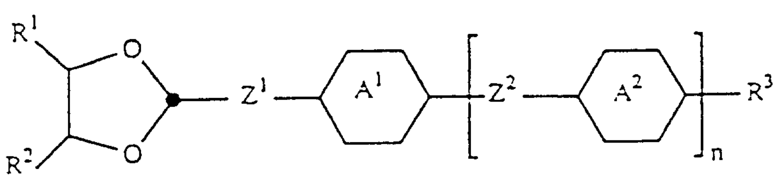

I

dans laquelle $R^3$ représente un groupe de formule générale

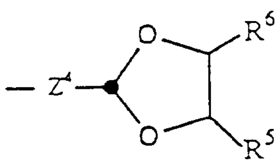

III

n, $R^1$, $R^2$, $R^5$, $R^6$, $Z^1$, $Z^2$, $Z^4$ et les cycles $A^1$ et $A^2$ sont tels que définis dans la revendication 1.

**4.** Composés possédant l'activité optique selon une des revendications 1 à 3, de formule générale

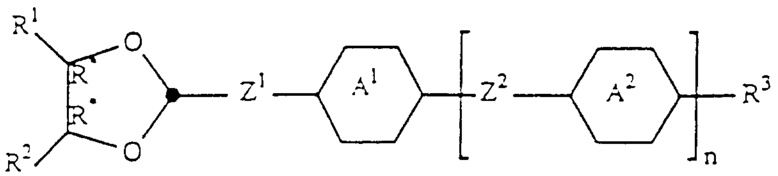

IA

dans laquelle $R^3$ représente un groupe de formule générale

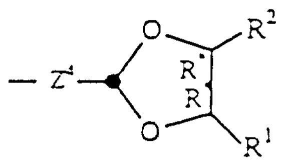

IIIA

$R^1$, $R^2$, $Z^1$, $Z^2$, $Z^4$ et les cycles $A^1$ et $A^2$ sont tels que définis dans la revendication 2.

**5.** Composés possédant l'activité optique selon revendication 1 ou 2, caractérisés en ce que l'un des cycles $A^1$, $A^2$ et $A^3$ est un cycle 1,4-phénylène non substitué ou substitué par des halogènes, des groupes cyano et/ou méthyle ou un cycle pyridine-2,5-diyle, pyrimidine-2,5-diyle, trans-1,4-cyclohexylène, trans-1,3-dioxanne-2,5-diyle ou naphtalène-2,6-diyle, $A^2$ pouvant également consister en un cycle 4,4'-biphénylène, les autres cycles, parmi $A^1$, $A^2$ et $A^3$, consistant, indépendamment l'un de l'autre, en cycles 1,4-phénylène non substitués ou substitués par des halogènes, des groupes cyano et/ou méthyle ou en cycles trans-1,4-cyclohexylène.

**6.** Composés possédant l'activité optique selon revendication 5, caractérisés en ce que $A^1$, $A^2$ et $A^3$, indépendamment les uns des autres, sont des cycles 1,4-phénylène non substitués ou substitués par des halogènes, des groupes cyano et/ou méthyle, ou des cycles trans-1,4-cyclohexylène, l'un des cycles $A^1$, $A^2$ et $A^3$ pouvant également consister en un cycle pyrimidine-2,5-diyle, l'un des symboles $Z^2$ et $Z^3$ représente $-(CH_2)_4-$, $-(CH_2)_3-O-$, $-O-(CH_2)_3-$ ou la forme trans de $-CH=CH-CH_2-CH_2-$, $-CH_2-CH_2-CH=CH-$, $-CH=CH-CH_2-O-$ ou $-O-CH_2-CH=CH-$ et l'autre représente un liaison covalente simple.

**7.** Composés possédant l'activité optique selon une des revendications 1 à 6, caractérisés par les formules générale

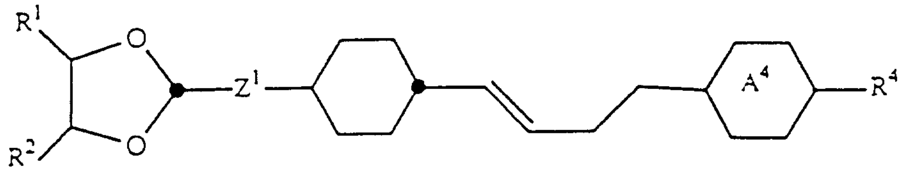

I-5

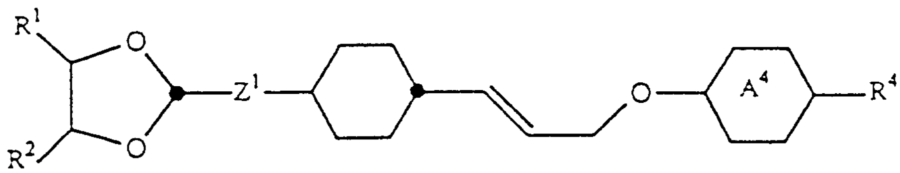

I-6

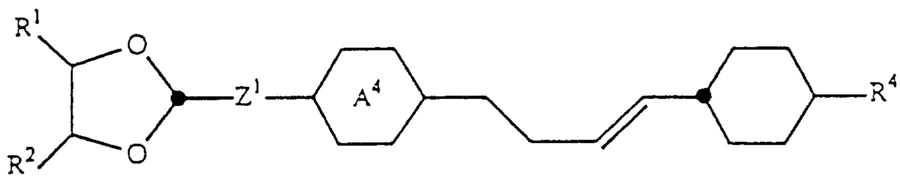

I-7

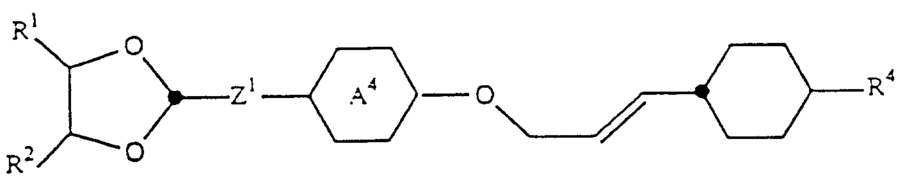

I-8

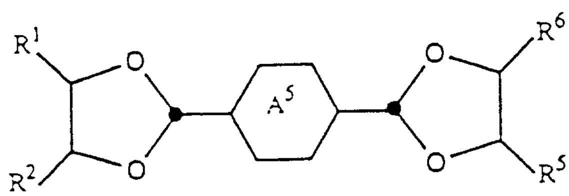

I-9

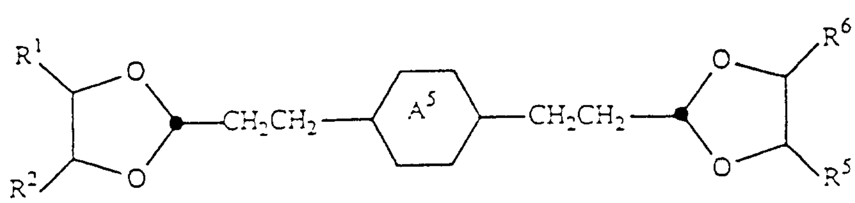

I-10

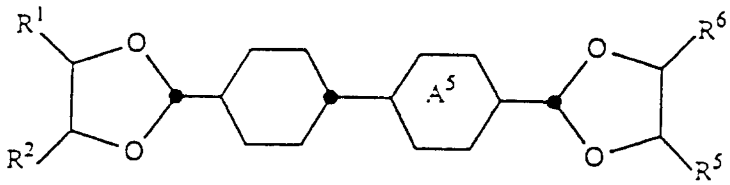

I-11

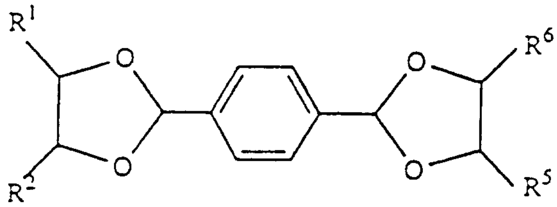

I-12

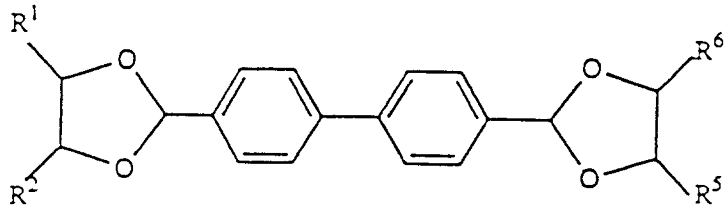

I-13

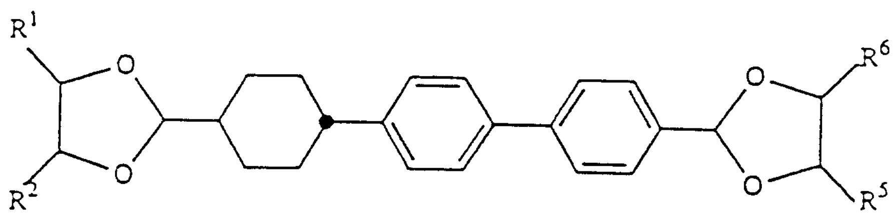

I-14

dans lesquelles $R^1$, $R^2$ et $R^4$ ont les significations indiquées dans la revendication 1 ; $R^5$ et $R^6$ ont les significations indiquées dans la revendication 1, de préférence $R^5$ a la même signification et, sur l'atome de carbone voisin, la même configuration que $R^1$, et $R^6$ a la même signification et, sur l'atome de carbone voisin, la même configuration que $R^2$ ; les cycles $A^4$ et $A^5$ sont des cycles 1,4-phénylène non substitués ou substitués par des halogènes, des groupes cyano et/ou méthyle, ou des cycles trans-1,4-cyclohexylène, $A^5$ pouvant également consister en un cycle 4,4'-biphénylène.

**8.** Composés possédant l'activité optique selon une des revendications 1, 2 et 5 à 7, caractérisés en ce que $R^4$ représente un halogène, un groupe cyano, trifluorométhyle, trifluorométhoxy, alkyle, alcényle, alcoxy, alcényloxy, 1-(alcoxycarbonyl)éthoxy ou 1-(alcoxycarbonyl)éthoxycarbonyle.

**9.** Composés possédant l'activité optique selon une des revendications 1, 2 et 5 à 8, caractérisés en ce que $R^4$ contient 1 à 12, de préférence 1 à 7 atomes de carbone.

**10.** Composés possédant l'activité optique selon une des revendications 1 à 9, caractérisés en ce que $R^1$ et $R^2$ représentent des groupes alcoxycarbonyle en C2-C7 ou bien $R^1$ et $R^2$ représentent des groupes méthyle ou bien $R^1$ et $R^2$ représentent des groupes phényle ou bien $R^1$ représente l'hydrogène et $R^2$ un groupe méthyle ou phényle, $R^5$ a les significations de $R^1$, $R^6$ les significations de $R^2$ et les atomes de carbone asymétriques ont la configuration relative indiquée dans la revendication 2.

**11.** Mélange à cristaux liquides contenant une matière de support à cristaux liquides et un ou plusieurs composés possédant l'activité optique selon les revendications 1 à 10.

**12.** Utilisation des mélanges à cristaux liquides selon revendication 9 pour des applications optiques ou électro-optiques.